# EUROPEAN PATENT APPLICATION

(11) **EP 2 763 066 A2**
(43) Date of publication of application: **06.08.2014**
(21) Application number: 14151706.0
(22) Date of filing: 20.01.2014
(51) Int. Cl.: G06F 19/00

(54) **Glucose meter**

(30) Priority: 25.01.2013 JP 2013012519
(71) Applicant: Terumo Kabushiki Kaisha, Tokyo 151-0072 (JP)
(72) Inventor: Nagasawa, Yasushi, Ashigarakami-gun, Kanagawa 259-0151 (JP); Tsuchiya, Yumiko, Chuo-ku, Tokyo (JP); Oohashi, Hirotaka, Chuo-ku, Tokyo (JP); Nomura, Takafumi, Ashigarakami-gun, Kanagawa 259-0151 (JP)
(74) Representative: TBK

(57) **Abstract**

A glucose meter has a postprandial alarm function for informing a patient of a fact that it is the postprandial glucose level measurement time. As it is detected that the current time obtained from an RTC reaches an alarm rumbling timing stored in an alarm timing memory, the I/O controller causes an alarm (buzzer) to rumble and sets a postprandial flag of a postprandial flag memory to a logic value "TRUE" for a predetermined time after the alarm rumbling timing. The postprandial flag is recorded on the glucose level table along with the glucose level measured by a glucose level measurement unit.

## Description

### BACKGROUND

### Technical Field

The present invention relates to a glucose meter capable of displaying a measured glucose level on a display screen.

### Related Art

In recent years, the number of patients suffering from diabetes rapidly increases. Diabetes is caused when the pancreas does not produce enough insulin, or because cells do not respond to the insulin.

For curing diabetes, it is important to suppress occurrence of complications. Therefore, it is necessary to normally control the glucose level. For this purpose, a glucose monitoring system (hereinafter, referred to as a glucose meter) has been developed for helping a patient or a family to conveniently measure the glucose level at home. The applicant is dedicated to developing a small-sized self-measurable glucose meter.

The applicant has been filed a patent application relating to a glucose meter, JP 2011-64596 A, which is incorporated by reference herein in entirety.

### SUMMARY

As described above, a diabetes patient is necessary to control the glucose level within an adequate level by himself/herself in the ordinary life. In addition, it is necessary to visit a hospital on a regular basis to report a glucose level measurement result to a doctor and counsel a lifestyle factor. In particular, a doctor recommends that a patient who treats diabetes with some medications measure the glucose level several times a day in order to prevent any complication and control the glucose level within in an adequate level. According to a guideline of International Diabetes Federation (IDF), it is recommended that a patient measure a postprandial glucose level based on a fact that the postprandial glucose level relates to a complication risk.

In recent years, as electronic devices are developed, the glucose meter is not a simple measurement device, but is highly possibly an information tool capable of urging a patient to manage the glucose level consistently.

Accordingly, the inventors made diligent efforts to develop a high functional glucose meter by employing functions of computing and displaying an average of the glucose level for a patient, particularly, displaying glucose level data of a patient for adequately controlling the glucose level, displaying a graphic status of the glucose level of a patient, alarming a patient for urging measurement of the glucose level, and the like.

Meanwhile, since the glucose meter is employed by various types of patients, it is necessary to provide a simple and easy manipulation method unlike an information device such as a personal computer (PC). In particular, it is desirable to provide a glucose meter that can be manipulated even by a patient not experienced in an information device, an old patient, a patient who suffers from a visual disorder, or the like. However, if various functions are employed in the glucose meter, a manipulation of the glucose meter may become complicated. Specifically, it is not desirable, for example, to increase the number of manipulation buttons in order to add functions, or to make a hierarchy of the menu for calling a desired function deep.

There is a demand for a novel glucose meter configured to provide various contents such as a graphic display function of the glucose level or an alarm function for urging a patient to measure a postprandial glucose level without harming its original purpose, that is, for enabling anybody to simply measure a glucose level.

In view of the aforementioned problems, it is desirable to provide a glucose meter capable of allowing a patient to more reliably perform a postprandial glucose level measurement task and having both high performance and simplicity.

In order to solve the above problem, the glucose meter of the present invention includes: a measurement unit where a test strip for measuring a glucose level of a patient is installed; a glucose level measurement unit that obtains a predetermined signal from the measurement unit and measures a glucose level of the patient; and a glucose level table including a date/time field where date/time information regarding a date and a time for measuring the glucose level is recorded, a glucose level field where the measured glucose level is recorded, and a mealtime flag field where a mealtime flag for identifying whether the measured glucose level is a preprandial glucose level or a postprandial glucose level is recorded. In addition, the glucose meter further includes a time counting unit that generates the date/time information; an alarm timing memory that stores an alarm setting timing; and a display unit that displays information representing a fact that it is the alarm setting timing when a current time obtained from the time counting unit matches the alarm setting timing. Furthermore, an I/O controller records a logic value indicating a fact that the measured glucose level is a postprandial glucose level on the mealtime flag field when the glucose level measurement unit records the measured glucose level on the glucose level table as long as the current time is within a predetermined period of time from the alarm setting timing.

According to the present invention, it is possible to provide a glucose meter capable of allowing a patient to more reliably perform a postprandial glucose level measurement task with a simple manipulation and having both high performance and simplicity.

### BRIEF DESCRIPTION OF DRAWINGS

FIGS. 1A to 1C are exterior diagrams illustrating a glucose meter according to an embodiment of the invention;
FIG. 2 is a block diagram illustrating a hardware configuration of the glucose meter;
FIG. 3 is a block diagram illustrating a software function of the glucose meter;
FIG. 4 is a schematic diagram illustrating an exemplary field structure and records of a glucose level table;
FIG. 5 is a state diagram roughly illustrating a state transition of the glucose meter;
FIG. 6 is a state diagram illustrating a state transition starting from a glucose meter alarm rumbling state;
FIG. 7 is a flowchart roughly illustrating an operation flow of the glucose meter;
FIG. 8 is a flowchart illustrating an operation flow of the glucose meter under a contents mode;
FIG. 9 is a flowchart illustrating an operation flow of the glucose meter in an initial screen display process under the contents mode;
FIG. 10 is a flowchart illustrating an operation flow of the glucose meter in an exception process under the contents mode;
FIG. 11 is a flowchart illustrating an operation flow of the glucose meter in a memory contents display process under the contents mode;
FIG. 12 is a flowchart illustrating an operation flow of the glucose meter in a memory value display process under the contents mode;
FIG. 13 is a flowchart illustrating an operation flow of the glucose meter in an average value display process under the contents mode;
FIGS. 14A to 14F are schematic diagrams illustrating a change of the display screen in the memory contents display process under the contents mode;
FIG. 15 is a flowchart illustrating an operation flow of the glucose meter in an alarm rumbling process;
FIGS. 16A and 16B are flowcharts illustrating processes operated in the glucose meter regardless of an operation mode under any one of the contents mode, the measurement mode, or the setup mode at all times.
FIGS. 17A and 17B are diagrams illustrating a display screen of a postprandial alarm;
FIG. 18 is a flowchart illustrating an operation flow of the glucose meter under the setup mode;
FIG. 19 is a flowchart illustrating an operation flow of the glucose meter under the measurement mode;
FIG. 20 is a flowchart illustrating an operation flow of the glucose meter under the measurement mode;
FIG. 21 is a flowchart illustrating an operation flow of the glucose meter in the exception process under the measurement mode;
FIG. 22 is a flowchart illustrating an operation flow of the glucose meter in a glucose level recording process under the measurement mode;
FIG. 23 is a flowchart illustrating an operation flow of the glucose meter in a button area display process under the measurement mode;
FIG. 24 is a flowchart illustrating an operation flow of the glucose meter in a glucose level display process under the contents mode and the measurement mode;
FIGS. 25A and 25B illustrate a display screen displayed on a display unit of the glucose meter in the glucose level display process under the measurement mode;
FIGS. 26A to 26E are diagrams illustrating a variation of emoticons displayed on the display unit of the glucose meter in the glucose level display process under the contents mode and the measurement mode;
FIGS. 27A and 27B are diagrams illustrating a variation of the display screen displayed on the display unit of the glucose meter in the glucose level display process under the contents mode and the measurement mode;
FIGS. 28A and 28B illustrate a display screen under the measurement mode and a display screen under the contents mode; and
FIG. 29 is a table showing functions allocated to manipulation buttons.

### DETAILED DESCRIPTION

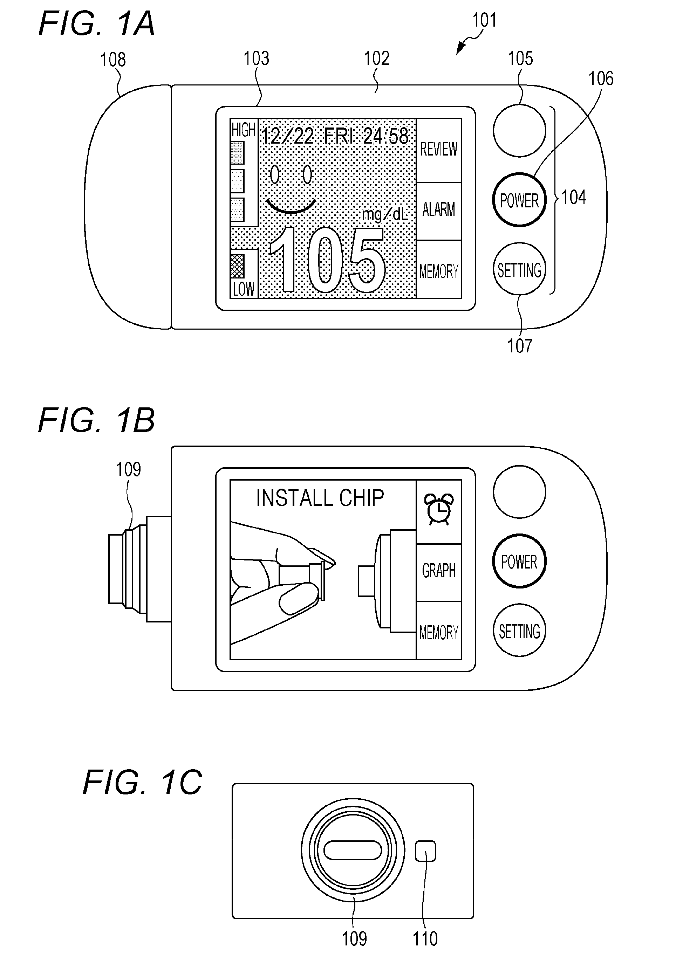

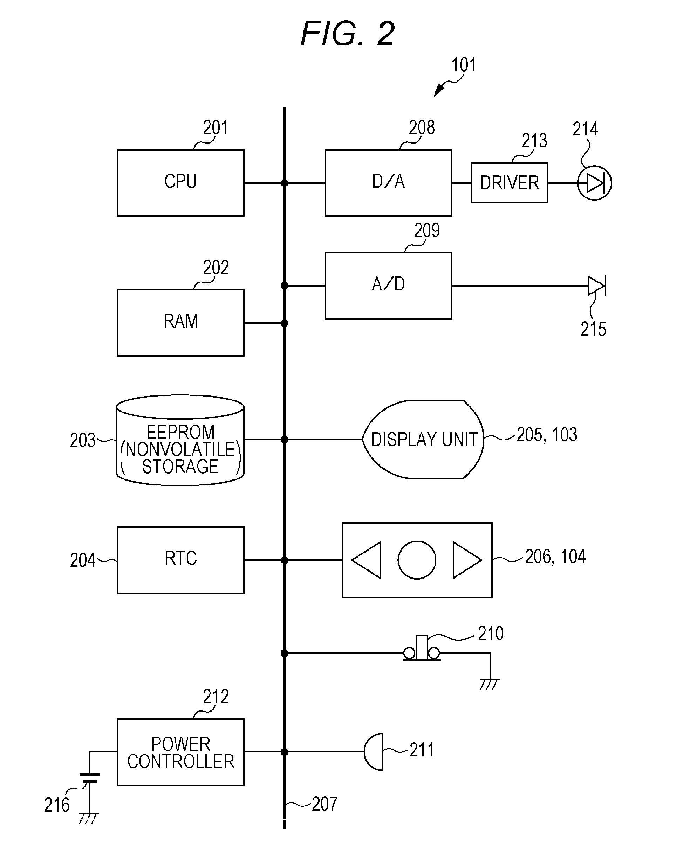

A glucose meter 101 according to an embodiment of the invention will be roughly described with reference to FIGS. 1A to 1C and 2.

FIGS. 1A to 1C show exterior diagrams illustrating the glucose meter 101 according to an exemplary embodiment of the invention.

The glucose meter 101 according to this embodiment has three manipulation buttons 104 including an upper button 105 as a first manipulation button, a middle button 106 as a second manipulation button, and a lower button 107 as a third manipulation button and a microswitch 210 (refer to FIG. 2) operated in synchronization with a cap 108 that protects an optical measurement unit 109.

As the cap 108 is removed, the process immediately advances to a measurement mode for measuring a glucose level.

If the middle button 106 is pressed and held for one or more seconds (hereinafter, this operation will be referred to as a "long press." In comparison, an operation of pressing the manipulation button 104 for a short time will be referred to a "short press"), the process advances to a contents mode for reading the glucose levels measured and stored in the past.

As a long press is performed for the lower button 107, the process advances to a setup mode for changing various setup values of the glucose meter 101.

The glucose meter 101 is provided with a so-called postprandial alarm function by which a countdown is performed for a predetermined time by setting an alarm. In addition, as the setting time elapses, an alarm sound is output using a buzzer 211.

Within one hour after the alarm rumbling, an elapsed time after the alarm rumbling is displayed on the liquid crystal display 103.

In addition, within one hour after the alarm sound is output, a postprandial flag as an example of a mealtime flag is automatically applied to the measured glucose level.

The mealtime flag is a flag indicating whether or not the measured glucose level is a preprandial glucose level or a postprandial glucose level based on a logic value thereof. According to this embodiment, a "postprandial flag" is employed, in which the flag is asserted in a postprandial state. Conversely, a "preprandial flag" may also be employed, in which the flag is asserted in a preprandial state.

In the glucose meter 101 according to this embodiment, the upper button 105 has a function of switching whether or not the postprandial flag is applied under the measurement mode.

In addition, in the glucose meter 101, the upper button 105 has a function of setting a postprandial alarm under the contents mode.

That is, the upper button 105 has a function relating to a concept of "postprandial" under both the measurement mode and the contents mode.

The glucose meter 101 according to this embodiment has a measurement history display function by which the glucose levels measured in the past can be read in a reversed order one by one under the contents mode. In addition, the glucose meter 101 also has a function of displaying the highest glucose level and an average value display function of displaying an average morning glucose level, an average daytime glucose level, and an average evening glucose level out of the glucose levels measured within a recent month.

The measurement history display function and the average value display function switch in a seamless manner in response to a manipulation of the upper button 105 or the lower button 107 without displaying a menu.

In the glucose meter 101 according to this embodiment, under the measurement mode, the measured glucose level is displayed as a numerical value and is evaluated on a 5-grade basis. An emoticon corresponding to the evaluation result is displayed on a screen of the liquid crystal display 103. Here, a background color of the screen is classified and displayed on a 5-grade basis. Furthermore, 5-grade colors are displayed on one side of the screen in a scale format, and a background color matching this scale is connected thereto and is displayed in a tab shape.

In the glucose meter 101 according to this embodiment, the measured glucose level or the past glucose level is displayed as a numerical value under the measurement mode and the contents mode. In addition, the glucose level is evaluated on a 5-grade basis, and the emoticon corresponding to the evaluation result is displayed on the screen of the liquid crystal display 103. It is noted that the background color of the screen is classified and displayed on a 5-grade basis. The classification and the emoticon can switch depending on a setting.

In the glucose meter 101 according to this embodiment, each of the measurement mode, the contents mode, and the setup mode is allocated to each of three manipulation buttons 104. Therefore, it is possible to implement multiple functions using a minimum number of manipulation buttons 104.

### Exterior

An exterior of the glucose meter 101 will be described with reference to FIGS. 1A to 1C.

The glucose meter 101 has a liquid crystal display 103 capable of color display on a surface of a casing 102 and a manipulation button 104 provided in the right edge of the liquid crystal display. The manipulation button 104 includes a membrane switch or a touch panel button known in the art.

The manipulation button 104 is provided in the outside of the liquid crystal display 103 and has the upper button 105, the middle button 106, and the lower button 107.

The upper button 105 is a button for upwardly moving a cursor displayed on the liquid crystal display 103. The lower button 107 is a button for downwardly moving the cursor. In addition, the middle button 106 is a button for instructing "EXECUTE," "ENTER," and the like.

If a short press is performed for the upper button 105 under the measurement mode, the upper button 105 serves as a "postprandial button" and records a postprandial flag described below on a corresponding record of the glucose level table 306.

If a long press is performed for the middle button 106, the glucose meter is powered on or off.

If a long press is performed for the lower button 107, the glucose meter enters a setup mode for changing miscellaneous setup values of the glucose meter 101.

An optical measurement unit 109 protected by a removable cap 108 is provided in one short side of the casing 102 of the glucose meter 101. A protrusion (not shown) is provided in the inside of the cap 108. As this protrusion meshes with a hollow 110 provided in the casing 102, a microswitch 210 (refer to FIG.

### 2) embedded in the casing 102 is turned off.

In a deactivated state, the glucose meter 101 operated using a battery (not shown) enters a power saving mode for reducing a power consumption.

If a long press is performed for the middle button 106 of the glucose meter 101 under a power saving mode, a predetermined menu screen is displayed on the liquid crystal display 103, and the process advances to the contents mode.

If the microswitch 210 is turned on by removing the cap 108 of the glucose meter 101 under the setup mode, a predetermined guide screen is displayed on the liquid crystal display 103, and the process advances to the measurement mode.

If a long press is performed for the lower button 107 of the glucose meter 101 under the setup mode, a predetermined menu screen is displayed on the liquid crystal display 103, and the process advances to the setup mode.

In the following description, the power saving mode state will be referred to as a "power-off state," and the measurement mode and the setup mode will be collectively referred to as a "power-on state."

It is noted that, when the process advances to the measurement mode by removing the cap 108 as illustrated in FIG. 1B, a chip installation guide illustration is displayed on the liquid crystal display 103 in order to measure the glucose level immediately.

### Hardware

FIG. 2 is a block diagram illustrating a hardware configuration of the glucose meter 101.

The glucose meter 101 has a microcomputer known in the art, including a central processing unit (CPU) 201, a random access memory (RAM) 202, an electrically erasable programmable read-only memory (EEPROM) 203, a real-time clock (RTC) 204 as a time counting unit for outputting date/time information, a display unit 205 as a liquid crystal display 103, and a manipulation unit 206 as a manipulation button 104 connected via a bus 207.

To the bus 207, a digital-analog (D/A) converter 208, an analog-digital (A/D) converter 209, a microswitch 210, a buzzer 211, and a power controller 212 are further connected.

A driver 213 is connected to the D/A converter 208, and an LED 214 is further connected to the driver 213. Since it is necessary to irradiate light onto a test strip 305 of the measurement chip with a suitable strength, the LED 214 is controlled to emit light based on strength data stored in the EEPROM 203.

A photodiode 215 is connected to the A/D converter 209.

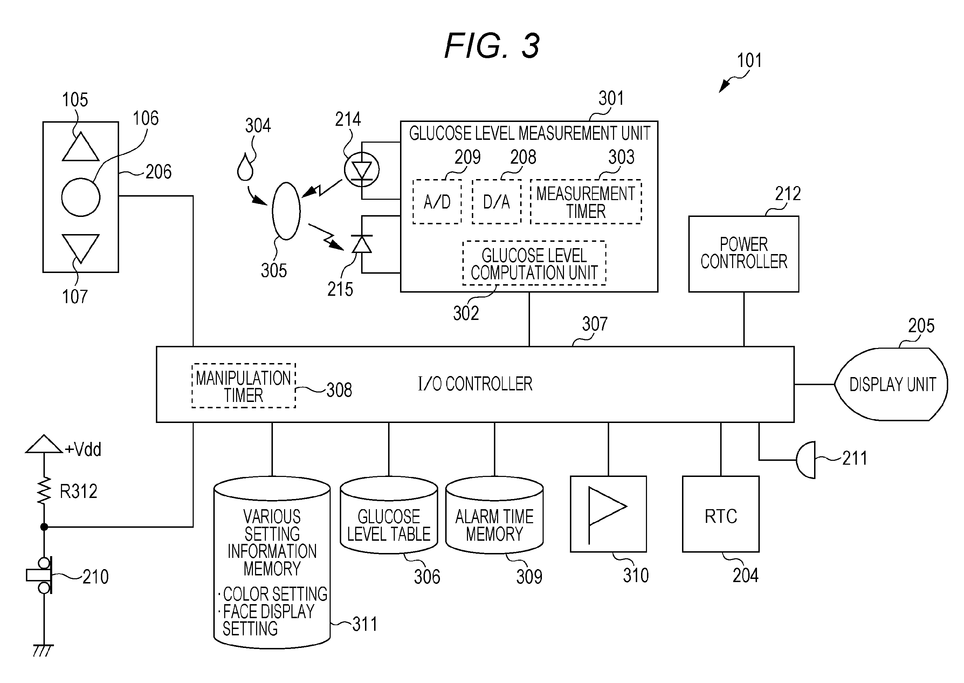

The photodiode 215 receives light of the LED 214 reflected on the test strip 305 (refer to FIG. 3) and generates an electric current depending on a strength of the light. This electric current is converted into numerical data using the A/D converter 209. In addition, this numerical data is subjected to a predetermined computation process and is recorded on a predetermined area of the RAM 202 and the EEPROM 203.

The LED 214 and the photodiode 215 are stored in the optical measurement unit 109.

The RTC 204 is an integrated circuit (IC) that provides a temporary data output function known in the art.

In the glucose meter 101 according to this embodiment, the date/time information regarding time of measuring the glucose level is recorded on the glucose level table 306 along with the glucose level (refer to FIG. 3).

Various screens are displayed on the display unit 205 based on a program stored in the EEPROM 203 and executed by the CPU 201. The display screens will be described below in detail.

The buzzer 211 is usually used to notify an operator of completion of the glucose level measurement, an alarm sound described below, or an error message.

The power controller 212 connected to the battery 216 controls supply of power in the entire glucose meter 101. Since the glucose meter 101 has the RTC 204, the RTC 204 is necessary to operate at all times regardless of the power-on state or the power-off state. The glucose meter 101 is also necessary to detect a state change of the manipulation unit 206 and the microswitch 210 even in the power-off state, that is, under the power saving mode. In this regard, under the power saving mode, the power controller 212 stops supply of power to devices desired to operate only in the power-on state, such as the display unit 205, D/A converter 208, the driver 213, and the A/D converter 209. In addition, under the power saving mode, the power controller 212 decreases an oscillation frequency of a system clock (not shown) for operating the CPU 201.

### Functional Configuration

FIG. 3 is a block diagram illustrating software functions of the glucose meter 101.

As described above, the glucose meter 101 is an electronic device having a microcomputer known in the art as a main component. The microcomputer reads a program (now shown) stored in the EEPROM 203 and configures the functional blocks of FIG. 3.

The glucose level measurement unit 301 detects a change of an optical reflectance of the test strip 305 whose color changes depending on blood 304 of an examinee to measure the glucose level. It is noted that the glucose level measurement unit 301 includes a D/A converter 208, an A/D converter 209, a glucose level computation unit 302, and a measurement timer 303.

A basic structure for measuring a glucose level of the glucose meter 101 is similar to that of the related art. Hereinafter, a structure for measuring a glucose level will be described shortly.

A measurement chip (not shown) is installed in the optical measurement unit 109, and blood 304 of an examinee is absorbed in the measurement chip. A test strip 305 made of a porous membrane such as polyethersulfone is embedded in the measurement chip. In addition, as the blood 304 absorbed in the measurement chip reaches the test strip 305, it reacts with a reagent contained in the test strip 305 to develop a color. Then, the light emitted from the LED 214 which is a light-emitting element is irradiated onto the test strip 305, and a photodiode 215 which is a light-receiving element receives the reflection light from the test strip 305. Then, after a predetermined reaction time elapses, an analog light intensity signal obtained from the photodiode 215 is converted into a digital value using the A/D converter 209, and the digital value is converted into a glucose level.

It is noted that a structure for measuring a glucose level is not limited to the aforementioned optical measurement method that uses a color-developing reagent. Instead, a structure used in glucose level measurement in the related art, such as an electrochemical sensor method, may also be employed.

The measurement timer 303 is a timer for counting a predetermined reactive time elapsing after blood 304 is absorbed in the test strip 305. In the glucose meter 101 of this embodiment, for example, a countdown of the measurement timer 303 is set to 9 seconds.

The glucose level computation unit 302 performs a predetermined computation process to compute a glucose level based on the data indicating the intensity of the reflection light obtained from the photodiode 215 using the A/D converter 209 when the measurement timer 303 counts the reactive time.

The RTC 204 outputs the date/time information. The RTC 204 is supplied with power even in a power-off state for accurately outputting the date/time information.

The glucose level table 306 is a table where the measurement date/time, the measurement result of the glucose level, and a postprandial flag described below are recorded. The glucose level table 306 is provided in a non-volatile storage such as the EEPROM 203.

The manipulation unit 206 corresponds to the manipulation button 104 described above. The display unit 205 corresponds to the liquid crystal display 103 described above.

The I/O controller 307 records the glucose level data obtained from the glucose level measurement unit 301 and the measurement date/time obtained from the RTC 204 on the glucose level table 306. In addition, it makes the measured glucose level being displayed on the display unit 205 or performs various data processings in response to manipulation of the manipulation unit 206.

The microswitch 210 is also connected to the I/O controller 307. One end of the microswitch 210 is pulled up using a resistor R312. The I/O controller 307 detects whether the cap 108 is installed in or removed from the casing 102 of the glucose meter 101 by detecting a change of the electric potential of the microswitch 210.

Furthermore, the I/O controller 307 is provided with the manipulation timer 308. The manipulation timer 308 is reset in response to a state change of the manipulation unit 206, the microswitch 210, and the glucose level measurement unit 301 and performs a countdown, for example, for 2 minutes at maximum. If it is detected that the manipulation timer 308 counts 2 minutes, the I/O controller 307 determines that time runs out, and the process advances to the power saving mode.

If a short press is performed for the upper button 105 while the menu screen of the contents mode is displayed, the I/O controller 307 obtains the current date/time information from the RTC 204 and causes the buzzer 211 to rumble after two hours (alarm rumbling). In this case, the timing of rumbling the alarm is stored in the alarm timing memory 309. The alarm timing memory 309 is provided in the EEPROM 203 which is a non-volatile memory because it is necessary to continuously store the alarm rumbling timing even under the power saving mode.

The postprandial flag memory 310 is a memory area provided in the EEPROM 203 which is a non-volatile storage for storing a postprandial flag state. The postprandial flag state stored in the postprandial flag memory 310 is changed by manipulating the upper button 105 under the measurement mode and is changed within an hour after the alarm rumbling or other time periods.

A miscellaneous setup information memory 311 is a memory area provided in the EEPROM 203 which is a non-volatile storage for storing miscellaneous setup information of the glucose meter 101. The miscellaneous setup information memory 311 includes a color setting of the display unit 205 and an emoticon display setting.

It is noted that the alarm timing memory 309, the postprandial flag memory 310, and the miscellaneous setup information memory 311 may be provided in the RAM 202 where the stored contents are backed up by a battery (battery-backed memory).

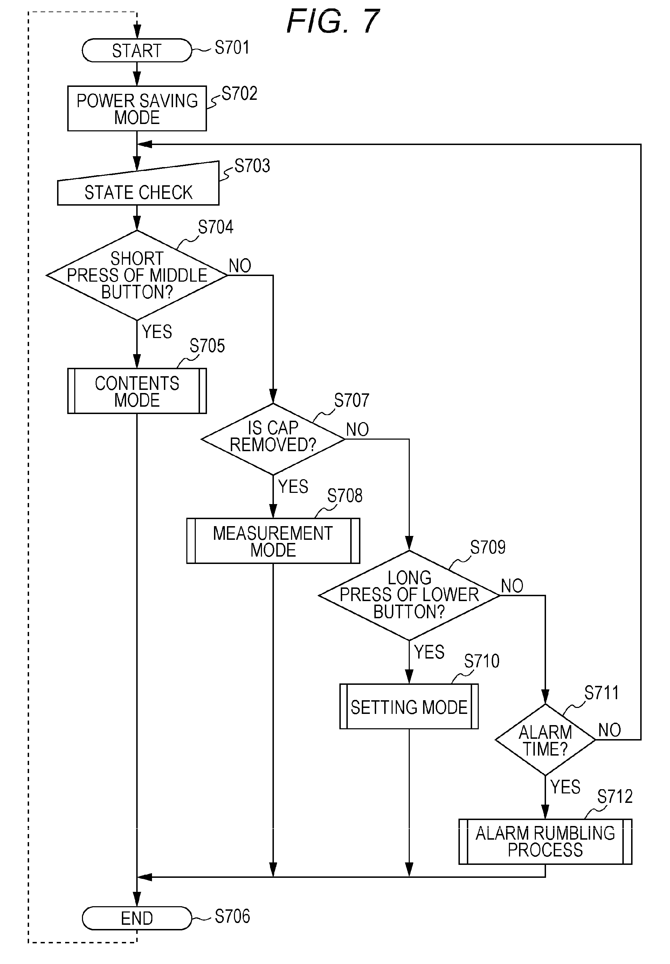

The I/O controller 307 detects a state change of the manipulation unit 206 and the microswitch 210 and implements a function corresponding to the state change. Such functions will be described in detail with reference to the flowcharts of FIG. 7 and the subsequent drawings.

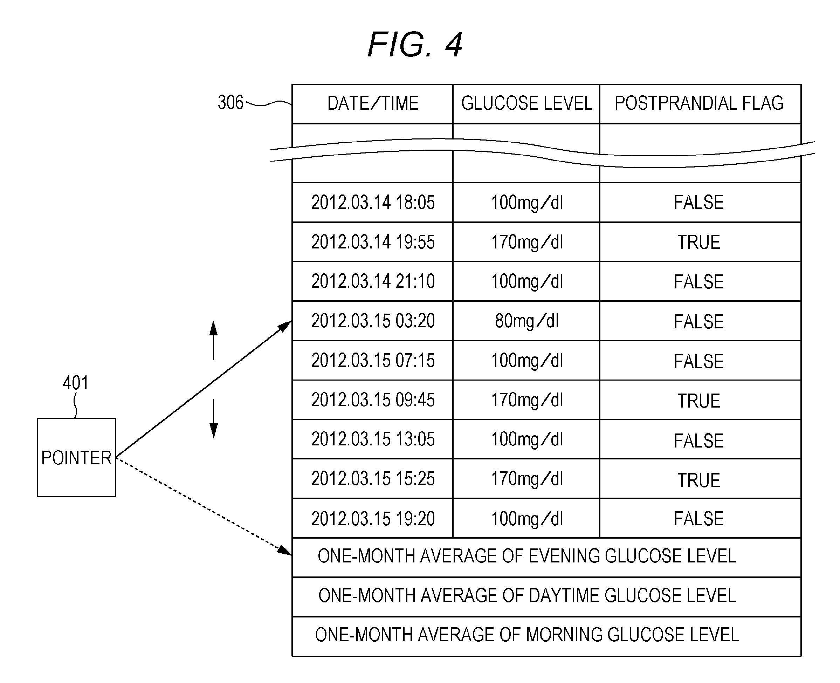

FIG. 4 is a schematic diagram illustrating an example of a field structure and records of the glucose level table 306.

A date/time field stores date/time information regarding the time of measuring the glucose level.

A glucose level field stores the measured glucose level.

A postprandial flag field stores a postprandial flag indicating that the measured glucose level is a postprandial glucose level by performing a short press for the upper button 105 when the glucose level is measured.

In the glucose level table 306, the glucose levels are recorded in chronological order. In addition, in the terminating end of the glucose level table 306, a label area different from the record of the glucose level table 306 is provided. This label area stores information indicating "ONE-MONTH AVERAGE OF EVENING GLUCOSE LEVEL," "ONE-MONTH AVERAGE OF DAYTIME GLUCOSE LEVEL," and "ONE-MONTH AVERAGE OF MORNING GLUCOSE LEVEL."

If a memory value display function is executed under the contents mode described below, a record of the glucose level table 306 indicated by a pointer 401 provided in the RAM 202 is displayed on the display unit 205. When the pointer 401 indicates the label area, the one-moth average of the glucose level is displayed on the display unit 205.

### State Transition

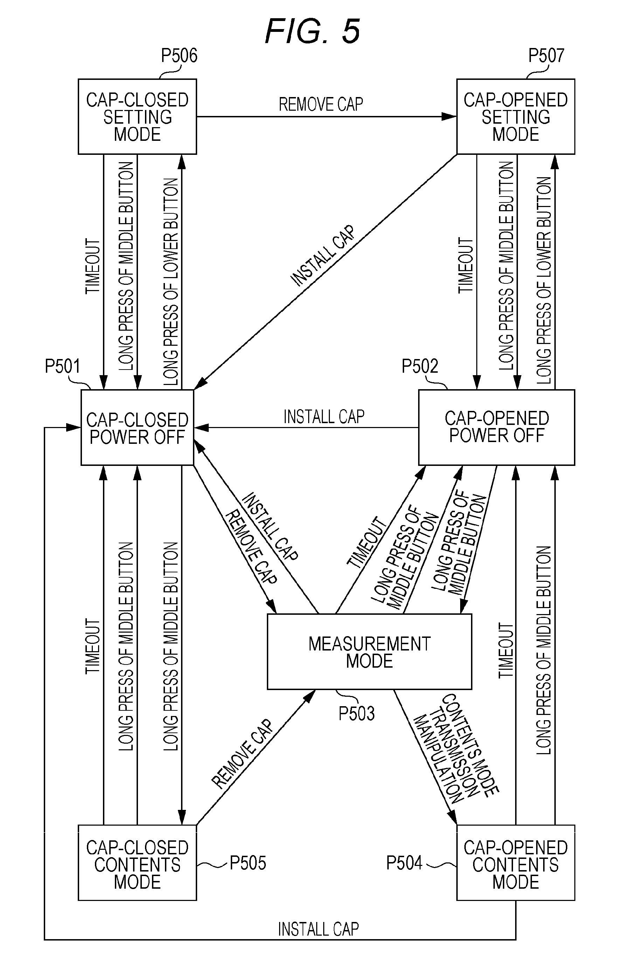

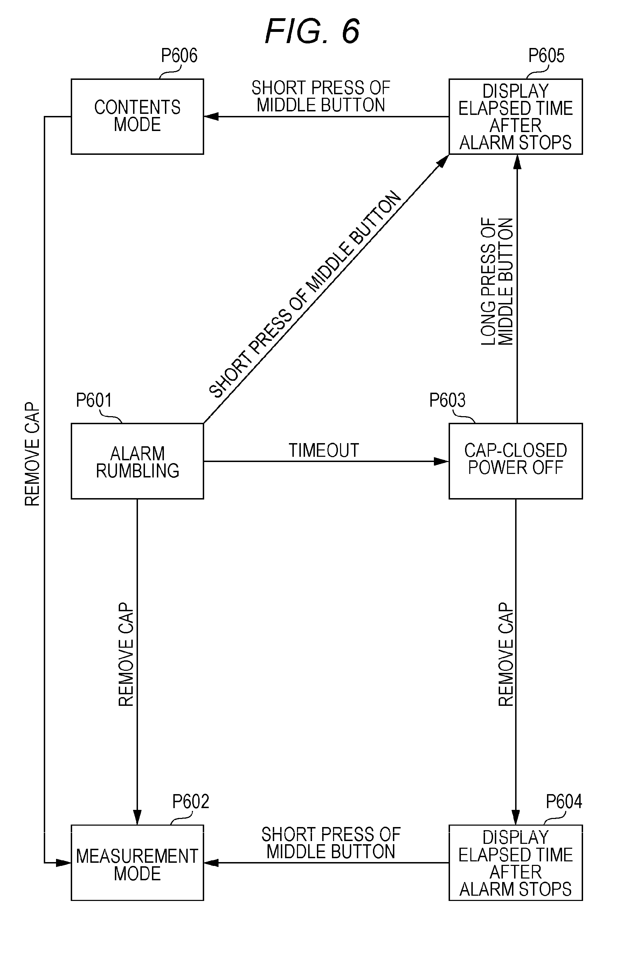

Next, a rough state change of the glucose meter 101 will be described with reference to the state diagrams of FIGS. 5 and 6.

First, a rough state change of the glucose meter 101 will be described with reference to FIG. 5.

FIG. 5 is a state diagram illustrating a rough state change of the glucose meter 101.

In an initial state, the glucose meter 101 is under a power saving mode. The power saving mode includes two types of power saving modes, that is, a cap-closed power-off (P501) in which the glucose meter 101 is powered off while the cap 108 is installed, and a cap-opened power-off (P502) in which the glucose meter 101 is powered off while the cap 108 is removed.

If the cap 108 is removed in the cap-closed power-off (P501) state, the glucose meter advances to the measurement mode (P503).

If the cap 108 is installed in the measurement mode (P503) state, the glucose meter advances to the cap-closed power-off (P501).

If time runs out by leaving the measurement mode (P503) state for a predetermined time (for example, two minutes) without any manipulation, or a long press is performed for the middle button 106, the glucose meter advances to the cap-opened power-off (P502).

If a long press is performed for the middle button 106 from the cap-opened power-off (P502) state, the glucose meter advances to the measurement mode (P503).

If a long press is performed for the middle button 106 in the cap-closed power-off (P501) state, the glucose meter advances to the cap-closed contents mode (P505).

If the cap 108 is removed in the cap-closed contents mode (P505) state, the glucose meter advances to the measurement mode (P503).

If time runs out by leaving the glucose meter without any manipulation for a predetermined time in the cap-closed contents mode (P505) state, or a long press is performed for the middle button 106, the glucose meter advances to the cap-closed power-off (P501).

If the manipulation button 104 is pressed for changing from the measurement mode (P503) state to the contents mode, the glucose meter advances to the cap-opened contents mode (P504).

If the cap 108 is installed in the cap-opened contents mode (P504) state, the glucose meter advances to the cap-closed power-off (P501).

If time runs out by leaving the glucose meter without any manipulation in the cap-opened contents mode (P504) state for a predetermined time, or a long press is performed for the middle button 106, the glucose meter advances to the cap-opened power-off (P502).

If a long press is performed for the lower button 107 in the cap-closed power-off (P501) state, the glucose meter advances to the cap-closed setup mode (P506).

If time runs out by leaving the glucose meter without any manipulation in the cap-closed setup mode (P506) state for a predetermined time, or a long press is performed for the middle button 106, the glucose meter advances to the cap-closed power-off (P501).

If the cap 108 is removed in the cap-closed setup mode (P506) state, the glucose meter advances to the cap-opened setup mode (P507).

If a long press is performed for the lower button 107 in the cap-opened power-off (P502) state, the glucose meter advances to the cap-opened setup mode (P507).

If time runs out by leaving the glucose meter without any manipulation in the cap-opened setup mode (P507) state for a predetermined time, or a long press is performed for the middle button 106, the glucose meter advances to the cap-opened power-off (P502).

If the cap 108 is installed in the cap-opened setup mode (P507) state, the glucose meter advances to the cap-closed power-off (P501).

If a manipulation is performed for "removal of cap 108" in the cap-closed power-off (P501) state and the cap-closed contents mode (P505) state, the glucose meter 101 advances to the measurement mode (P503) . That is, the operation of removing the cap 108 also serves as an instruction of immediate power-on and an instruction of starting the measurement of the glucose level.

If a manipulation is performed for "installation of cap 108" in the measurement mode (P503) state, the cap-opened contents mode (P504) state, and the cap-opened setup mode (P507) state, the glucose meter 101 advances to the cap-closed power-off (P501) . That is, the operation of installing the cap 108 is an immediate power-off instruction.

FIG. 6 is a state diagram illustrating a change of the glucose meter 101 starting from an alarm rumbling state. It is noted that it is assumed that the cap 108 is installed at the time of the alarm rumbling for convenient description purposes.

If the cap 108 is removed in the alarm rumbling (P601) state, the glucose meter advances to the measurement mode (P602).

If time runs out by leaving the glucose meter without any manipulation in the alarm rumbling (P601) state for a predetermined time, the glucose meter advances to the cap-closed power-off (P603).

If the cap 108 is removed in the cap-closed power-off (P603) state, the glucose meter advances to the alarm-stopped elapsed time display (P604) in which an elapsed time after the alarm stops is displayed on a screen.

If a short press is performed for the middle button 106 in the alarm-stopped elapsed time display (P604) state, the glucose meter advances to the measurement mode (P602).

If a short press is performed for the middle button 106 in the alarm rumbling (P601) state, the glucose meter advances to the alarm-stopped elapsed time display (P605) state.

If a long press is performed for the middle button 106 in the cap-closed power-off (P603) state, the glucose meter advances to the alarm-stopped elapsed time display (P605).

If a short press is performed for the middle button 106 in the alarm-stopped elapsed time display (P605) state, the glucose meter advances to the contents mode (P606).

If the cap 108 is removed in the contents mode (P606) state, the glucose meter advances to the measurement mode (P602).

The glucose meter 101 according to this embodiment is characterized in the postprandial alarm. Since the time allowed to measure the postprandial glucose level is limited, an alarm is set for a time considered as the postprandial measurement time in advance. In the postprandial alarm, as a setting timing is reached, the alarm rumbles, and the elapsed time is displayed on the liquid crystal display 103. As the elapsed time is displayed on the liquid crystal display 103, a patient may determine whether or not it is time suitable to measure a postprandial glucose level.

In addition, in the postprandial alarm, since the alarm is rumbled, and the postprandial flag is set to a logic value "TRUE." Therefore, it is not necessary to apply the postprandial flag by manipulating the upper button 105 when the glucose level is measured.

### Operation Flow

FIG. 7 is a flowchart illustrating a schematic operation flow of the glucose meter 101.

If the processing is initiated (S701) at the event of battery installation or the like, initially, the I/O controller 307 controls the power controller 212 to set the operation state of the glucose meter 101 to the power saving mode (S702).

Then, the I/O controller 307 checks states of the manipulation button 104, the microswitch 210, and the RTC 204 under the power saving mode (S703).

If a short press is performed for the middle button 106 (YES in S704), the I/O controller 307 sets the operation state of the glucose meter 101 to the contents mode and performs a process of the contents mode (S705). As the process of the contents mode is terminated, the I/O controller 307 terminates a series of processes (S706). Then, the process starts again from step S701, and in step S702, the glucose meter 101 sets the operation state to the power saving mode.

If a short press is not performed for the middle button 106 in step S704 (NO in S704), the I/O controller 307 checks a state of the microswitch 210. As a result, if it is checked that the cap 108 is removed (YES in S707), the I/O controller 307 sets the operation state of the glucose meter 101 to the measurement mode and performs a process of the measurement mode (S708). As the process of the measurement mode is terminated, the I/O controller 307 terminates a series of processes (S706). In addition, the process starts again from step S701, and in step S702, the glucose meter 101 sets the operation state to the power saving mode.

In step S707, if the cap 108 is not removed (NO in S707), the I/O controller 307 checks a state of the lower button 107. As a result, it is checked that a long press is performed for the lower button 107 (YES in S709), the I/O controller 307 sets the operation state of the glucose meter 101 to the setup mode and performs a process of the setup mode (S710). As the process of the setup mode is terminated, the I/O controller 307 terminates a series of processes (S706). In addition, the process starts again from step S701, and in step S702, the glucose meter 101 sets the operation state to the power saving mode.

If a long press is not performed for the lower button 107 in step S709 (NO in S709), the I/O controller 307 checks a state of the RTC 204. As a result, if it is checked that the current time reaches an alarm setting timing (YES in S711), the I/O controller 307 performs an alarm rumbling process (S712). As the alarm rumbling process is terminated, the I/O controller 307 terminates a series of processes (S706). In addition, the process starts again from step S701, and in step S702, the glucose meter 101 sets the operation state to the power saving mode.

If the current time does not reach the alarm setting timing in step S711 (NO in S711), the I/O controller 307 repeats the process again from step S703, and in the power saving mode state, checks states of the manipulation button 104, the microswitch 210, and the RTC 204 (S703).

### Contents Mode

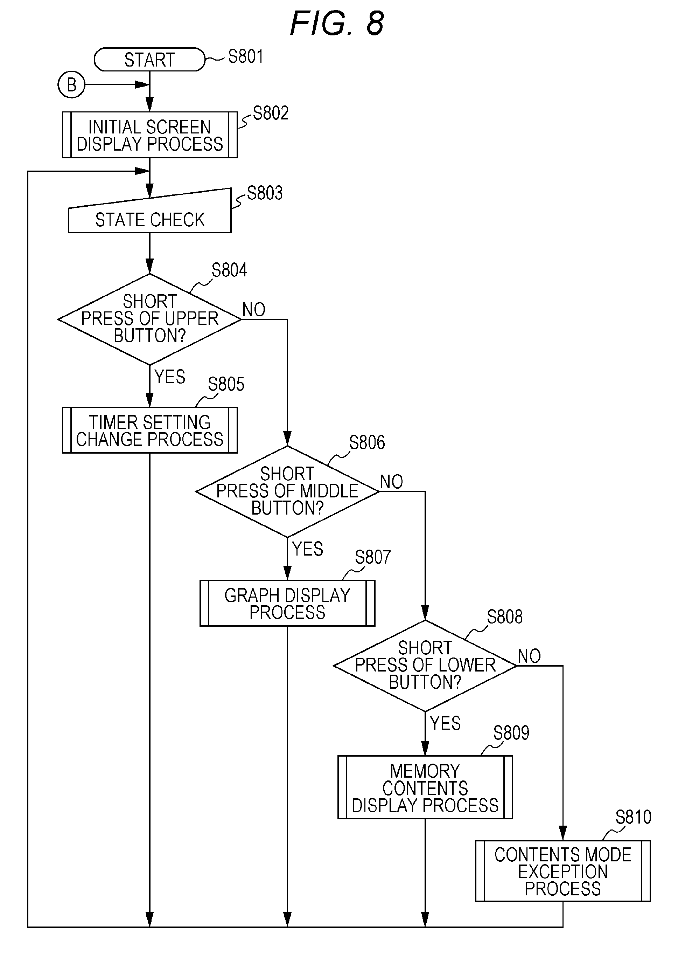

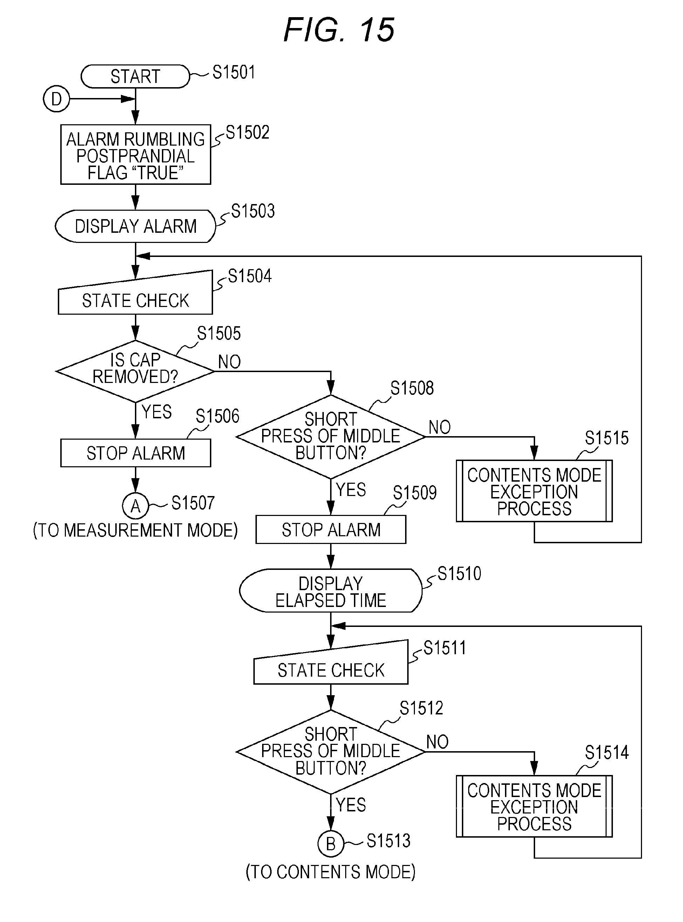

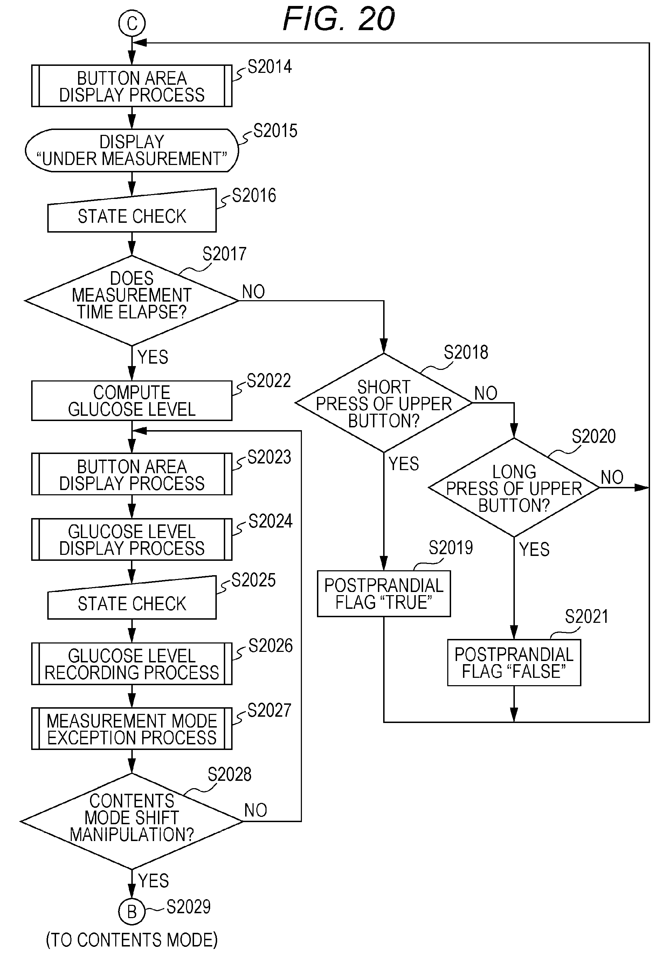

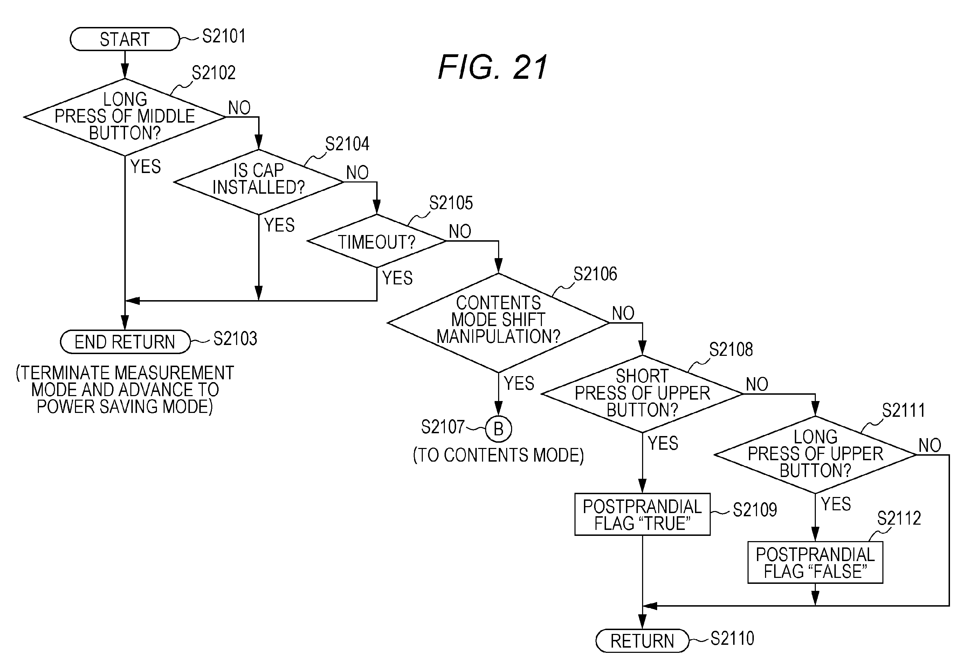

FIG. 8 is a flowchart illustrating an operation flow of the contents mode of the glucose meter 101. FIG. 8 corresponds to step S705 of FIG. 7 and is also executed from step S1513 of FIG. 15, step S2029 of FIG. 20, and step S2107 of FIG. 21 described below.

As the process starts (S801), initially, the I/O controller 307 executes an initial screen display process (S802).

Then, the I/O controller 307 checks states of the manipulation button 104 and the microswitch 210 (S803). As a result, if a short press is performed for the upper button 105 (YES in S804), the I/O controller 307 performs a timer setting change process (S805). As the timer setting change process is terminated, the I/O controller 307 checks the states of the manipulation button 104 and the microswitch 210 again (S803).

If a short press is not performed for the upper button 105 in step S804 (NO in S804), the I/O controller 307 checks a state of the middle button 106. As a result, it is checked that a short press is performed for the middle button 106 (YES in S806), the I/O controller 307 performs a graph display process (S807). If the graph display process is terminated, the I/O controller 307 checks the states of the manipulation button 104 and the microswitch 210 again (S803).

If a short press is not performed for the middle button 106 in step S806 (NO in S806), the I/O controller 307 checks a state of the lower button 107. As a result, it is checked that a short press is performed for the lower button 107 (YES in S808), the I/O controller 307 performs a memory contents display process (S809). As the memory contents display process is terminated, the I/O controller 307 checks the states of the manipulation button 104 and the microswitch 210 again (S803).

If a short press is not performed for the lower button 107 in step S808 (NO in S806), the I/O controller 307 performs a contents mode exception process (S809). As the contents mode exception process is terminated, the I/O controller 307 checks the states of the manipulation button 104 and the microswitch 210 again (S803).

### Contents Mode: Initial Screen Display Process

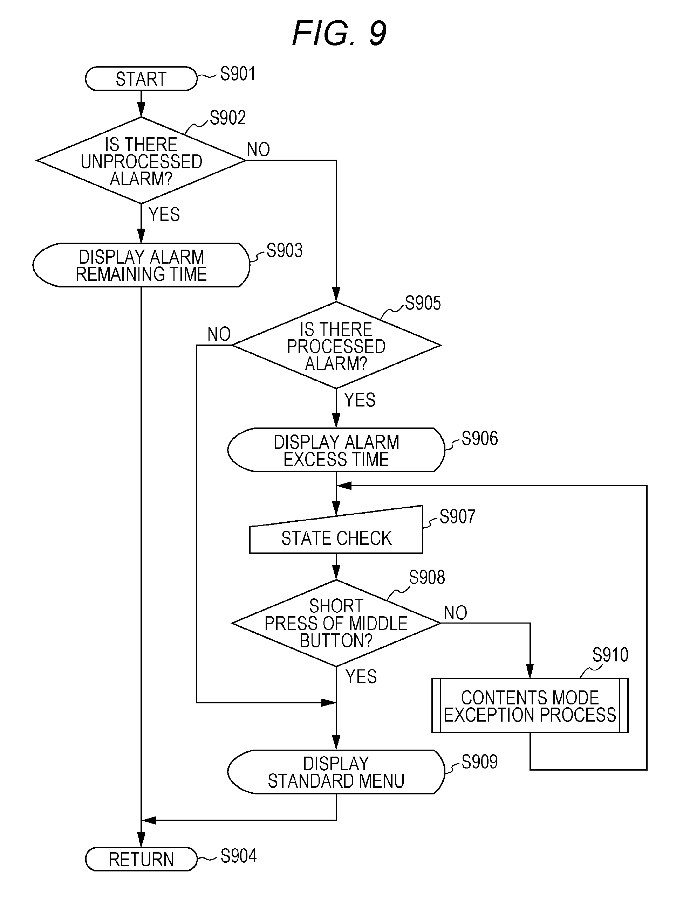

FIG. 9 is a flowchart illustrating an operation flow of the glucose meter 101 in the initial screen display process under the contents mode. FIG. 9 corresponds to step S802 of FIG. 8.

As the process starts (S901), the I/O controller 307 initially obtains current date/time information from the RTC 204 and references the alarm rumbling timing stored in the alarm timing memory 309 to determine whether two hours or shorter remains until the alarm rumbling timing, that is, whether or not the postprandial alarm has not been rumbled (S902). If it is determined that two hours or shorter remains until the alarm rumbling timing (YES in S902), the I/O controller 307 displays the remaining time of the postprandial alarm on the display unit 205 (S903), and a series of processes are terminated (S904).

If the alarm rumbling timing is not within two hours in step S902 (NO in S902), the I/O controller 307 determines whether or not one hour or shorter elapses after the alarm rumbling timing, that is, whether or not one hour elapses after the postprandial alarm rumbles (S905). If one hour or shorter elapses after the alarm rumbling timing (YES in S905), the I/O controller 307 displays the excess time of the postprandial alarm on the display unit 205 (S906), and checks the states of the manipulation button 104 and the microswitch 210 (S907). As a result, if a short press is performed for the middle button 106 (YES in S908), the I/O controller 307 displays a standard menu screen of the contents mode on the display unit 205 (S909), and a series of processes are terminated (S904).

If a short press is not performed for the middle button 106 in step S908 (NO in S908), the I/O controller 307 executes an exception process of the contents mode (S910) and checks the states of the manipulation button 104 and the microswitch 210 again (S907).

### Contents Mode: Exception Process

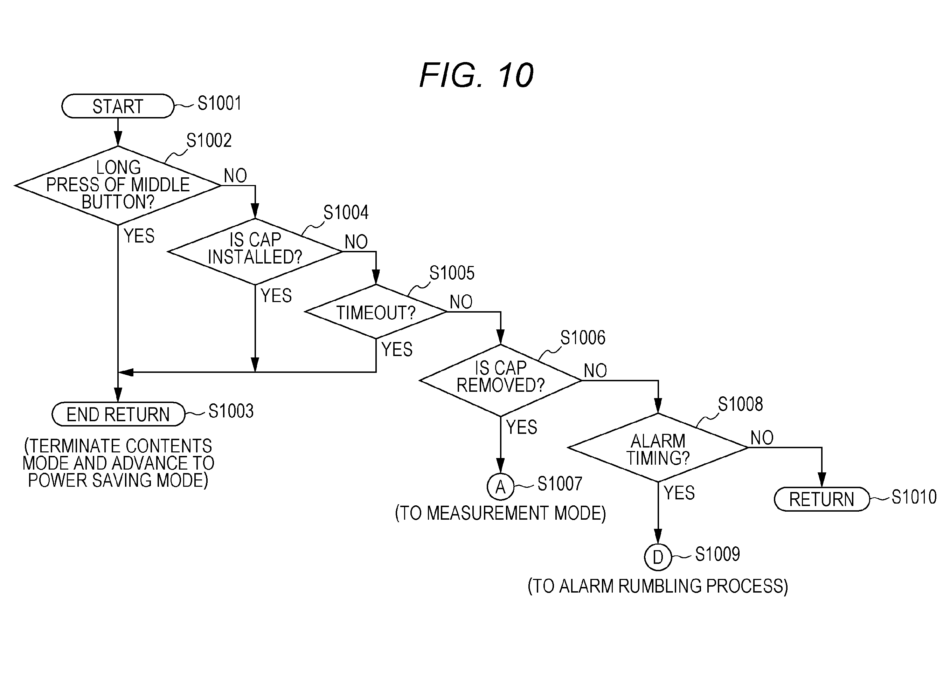

FIG. 10 is a flowchart illustrating an operation flow of the glucose meter 101 in the exception process under the contents mode. FIG. 10 corresponds to step S810 of FIG. 8 and step S910 of FIG. 9.

As the process starts (S1001), the I/O controller 307 checks whether or not a long press is performed for the middle button 106 (S1002). If a long press is performed for the middle button 106 (YES in S1002), the I/O controller 307 terminates the process of the contents mode (S1003). As a result, the glucose meter 101 advances to the power saving mode (S702 of FIG. 7).

If a long press is not performed for the middle button 106 in step S1002 (NO in S1002), the I/O controller 307 references the state of the microswitch 210 and checks whether or not the cap 108 is installed (S1004). If the cap 108 is installed (YES in S1004), the I/O controller 307 terminates the process of the contents mode (S1003). As a result, the glucose meter 101 advances to the power saving mode (S702 of FIG. 7).

If the cap 108 is not installed in step S1004 (NO in S1004), the I/O controller 307 references the manipulation timer 308 and checks whether or not the manipulation timer 308 reaches a timeout (S1005). If the manipulation timer 308 reaches a timeout (YES in S1005), the I/O controller 307 terminates the process of contents mode (S1003). As a result, the glucose meter 101 advances to the power saving mode (step S702 of FIG. 7).

If the manipulation timer does not reach a timeout in step S1005 (NO in S1005), the I/O controller 307 references a state of the microswitch 210 and checks whether or not the cap 108 is removed (S1006). If the cap 108 is removed (YES in S1006), the I/O controller 307 advances the process from the contents mode to the measurement mode (S1007). As a result, the glucose meter 101 advances to the measurement mode (step S708 of FIG. 7)

If the cap 108 is not removed in step S1006 (NO in S1006), the I/O controller 307 obtains the current date/time information from the RTC 204 and references the alarm rumbling timing stored in the alarm timing memory 309 to check whether or not the current time reaches the alarm rumbling timing (S1008).

If the current time reaches the alarm timing (YES in S1008), the I/O controller 307 advances the process from the contents mode process to the alarm rumbling process (S1009). As a result, the glucose meter 101 advances to the alarm rumbling process (step S712 of FIG. 7).

If the current time does not reach the alarm timing in step S1008 (NO in S1008), the I/O controller 307 terminates the exception process of the contents mode (S1010).

### Contents Mode: Memory Contents Display Process

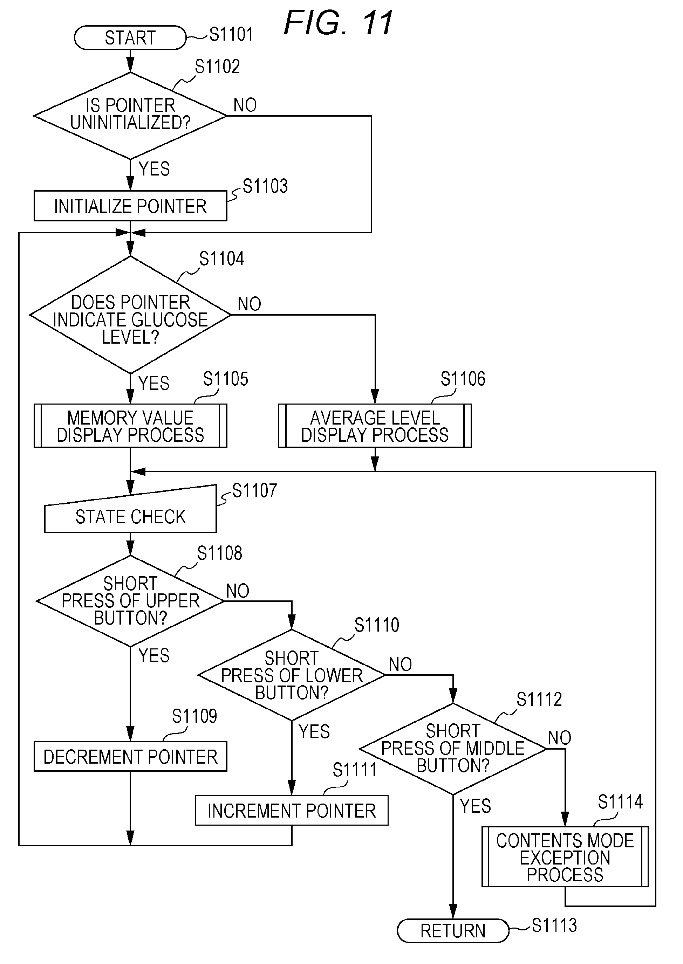

FIG. 11 is a flowchart illustrating an operation flow of the glucose meter 101 in the memory contents display process under the contents mode. FIG. 11 corresponds to step S809 of FIG. 8.

As the process starts (S1101), the I/O controller 307 checks whether or not the pointer 401 is initialized (S1102). If the pointer 401 is not initialized (YES in S1102), the I/O controller 307 initializes the pointer 401 (S1103).

If the pointer 401 is initialized in step S1103, or the pointer 401 is initialized in step S1102 (NO in S1102), the I/O controller 307 references the content of the pointer 401 and checks whether or not the pointer 401 indicates a glucose level record of the glucose level table 306 (S1104). If the pointer 401 indicates at the glucose level record (YES in S1104), the I/O controller 307 executes a memory value display process in which the content of the glucose level record is displayed (S1105).

If the pointer 401 does not point at the glucose level record in step S1104 (NO in S1104), the I/O controller 307executes an average value display process in which an average of the glucose levels for a recent month is displayed in response to an average value display instruction indicated by the pointer 401 (S1106).

After either the memory value display process of step S1105 or the average value display process of step S1106 is executed, the I/O controller 307 checks the states of the manipulation button 104, the microswitch 210, and the RTC 204 (S1107).

First, the I/O controller 307 checks whether or not a short press is performed for the upper button 105 (S1108). If a short press is performed for the upper button 105 (YES in S1108), the I/O controller 307 decrements the value of the pointer 401 S1109) and repeats the process from step S1104.

If a short press is not performed for the upper button 105 in step S1108 (NO in S1108), the I/O controller 307 checks whether or not a short press is performed for the lower button 107 (S1110). If a short press is performed for the lower button 107 (YES in S1110), the I/O controller 307 increments the value of the pointer 401 (S1111) and repeats the process from step S1104.

If a short press is not performed for the lower button 107 in step S1110 (NO in S1110), the I/O controller 307 checks whether or not a short press is performed for the middle button 106 (S1112). If a short press is performed for the middle button 106 (YES in S1112), the I/O controller 307 terminates the memory contents display process of the contents mode (S1113).

If a short press is not performed for the middle button 106 in step S1112 (NO in S1112), the I/O controller 307 executes the exception process of the contents mode (S1114) and checks the states of the manipulation button 104, the microswitch 210, and the RTC 204 again (S1107).

### Contents Mode: Memory Value Display Process

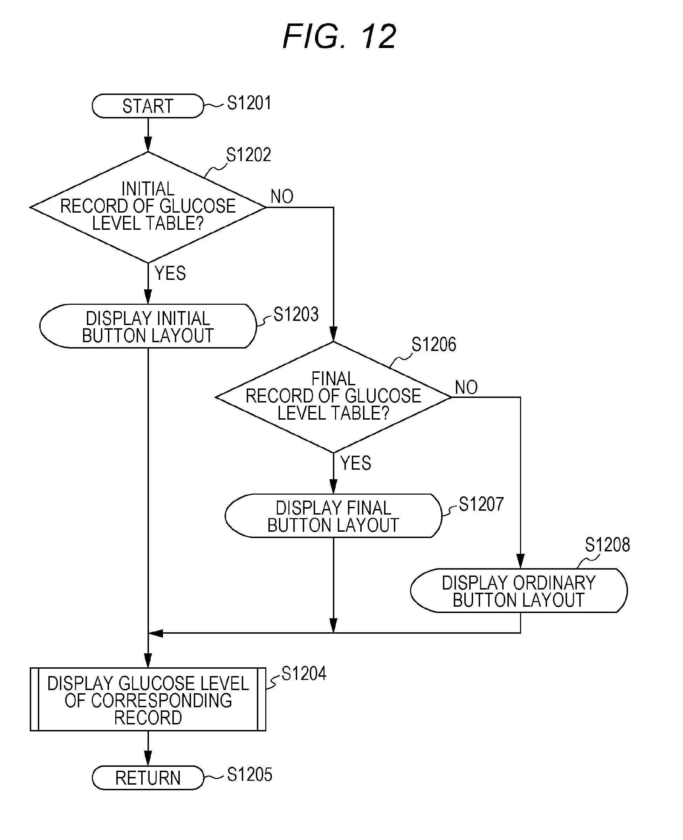

FIG. 12 is a flowchart illustrating an operation flow of the glucose meter 101 in the memory value display process under the contents mode. FIG. 12 corresponds to step S1105 of FIG. 11.

As the process starts (S1201), the I/O controller 307 checks whether or not the record indicated by the pointer 401 is an initial record of the glucose level table 306 (S1202). If the record indicated by the pointer 401 is the initial record of the glucose level table 306 (YES in S1202), the I/O controller 307 displays a button layout for the initial record on the display unit 205 (S1203). In addition, the glucose level of the record indicated by the pointer 401 is displayed on the display unit 205 (S1204), and the memory value display process is terminated (S1205).

If the record indicated by the pointer 401 is not the initial record of the glucose level table 306 in step S1202 (NO in S1202), the I/O controller 307 checks whether or not the record indicated by the pointer 401 is the last record of the glucose level table 306 (S1206). If the record indicated by the pointer 401 is the last record of the glucose level table 306 (YES in S1206), the I/O controller 307 displays a button layout for the last record on the display unit 205 (S1207). In addition, the glucose level of the record indicated by the pointer 401 is displayed on the display unit 205 (S1204), and the memory value display process is terminated (S1205).

If the record indicated by the pointer 401 is not the last record of the glucose level table 306 in step S1206 (NO in S1206), the I/O controller 307 displays a button layout for a typical record on the display unit 205 (S1208). In addition, the glucose level of the record indicated by the pointer 401 is displayed on the display unit 205 (S1204), and the memory value display process is terminated (S1205).

### Contents Mode: Average Value Display Process

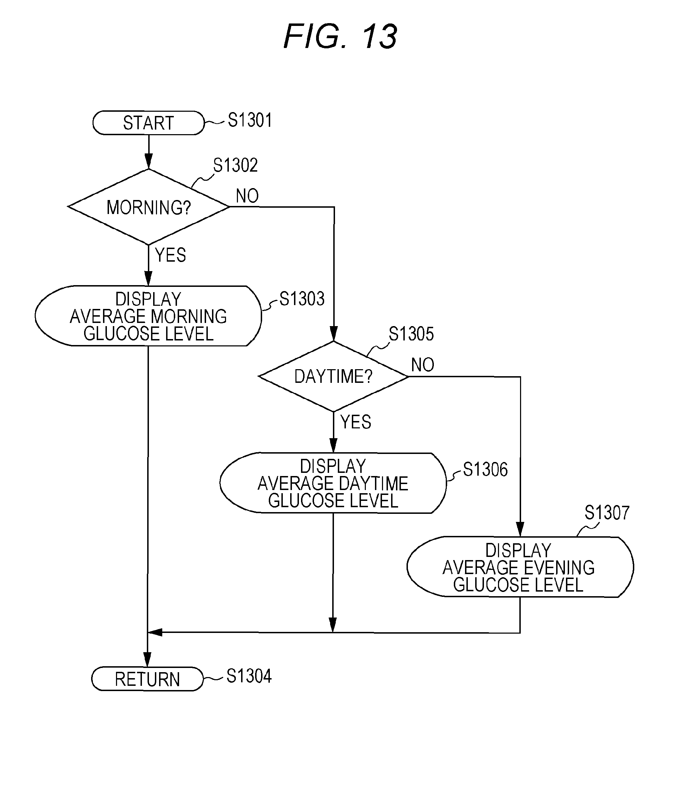

FIG. 13 is a flowchart illustrating an operation flow of the glucose meter 101 in the average value display process under the contents mode. FIG. 13 corresponds to step S1106 of FIG. 11.

As the process starts (S1301), the I/O controller 307 checks whether or not the label indicated by the pointer 401 is an instruction for displaying an average of the morning glucose level out of the glucose levels recorded on the glucose level table 306 (S1302). If the label indicated by the pointer 401 is an instruction for displaying an average of the morning glucose level (YES in S1302), the I/O controller 307 displays the average of the morning glucose level on the display unit 205 (S1303). In addition, the average value display process is terminated (S1304).

If the label indicated by the pointer 401 is not an instruction for displaying the average of the morning glucose level in step S1302 (NO in S1302), the I/O controller 307 checks whether or not the label indicated by the pointer 401 is an instruction for displaying the average of the daytime glucose level out of the glucose levels recorded on the glucose level table 306 (S1305). If the label indicated by the pointer 401 is the instruction for displaying the average of the daytime glucose level (YES in S1305), the I/O controller 307 displays the average of the daytime glucose level on the display unit 205 (S1306). In addition, the average value display process is terminated (S1304).

If the label indicated by the pointer 401 is not the instruction for displaying the average of the daytime glucose level in step S1305 (NO in S1305), the I/O controller 307 displays the average of the evening glucose level on the display unit 205 (S1307). In addition, the average value display process is terminated (S1304).
Contents Mode: Display Screen in Memory Contents Display Process and Average Value Display Process

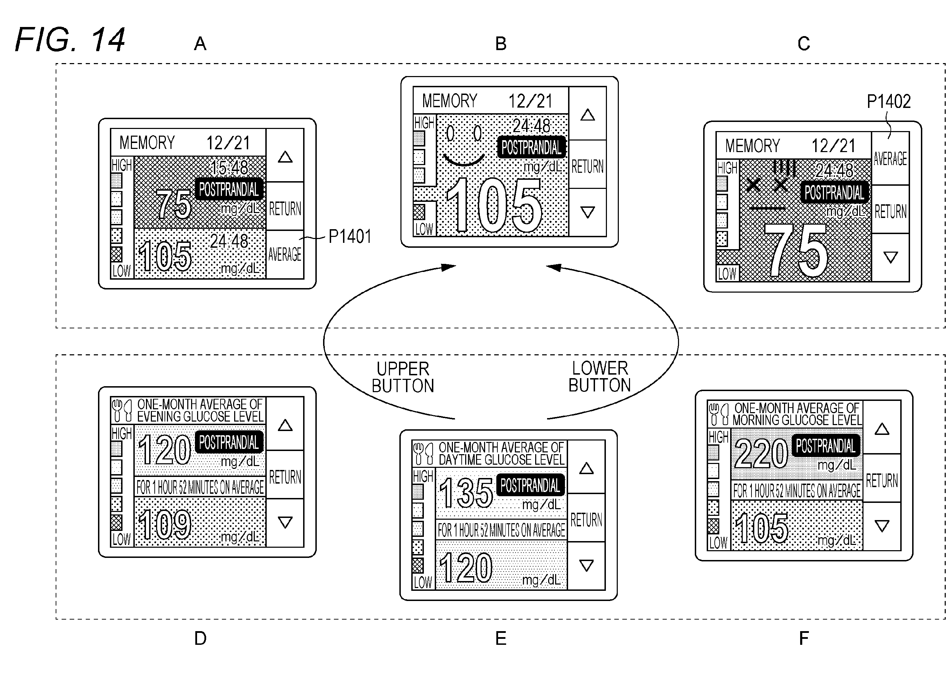

FIGS. 14A to 14F are schematic diagrams illustrating a change of the display screen in the memory contents display process under the contents mode.

If a short press is performed for the lower button 107 (step S808 of FIG. 8), and the memory contents display process is selected (S809 of FIG. 8) after a menu screen is displayed under the contents mode (FIG. 8) (step S903 or S909 of FIG. 8), the I/O controller 307 displays the previous contents indicated by the pointer 401 on the display unit 205.

A guidance for the functions of the manipulation button 104 is displayed on the right side of the screen in FIGS. 14A to 14F.

If the pointer 401 indicates the initial record of the glucose level table 306 (step S1202 of FIG. 12), the I/O controller 307 displays the screen of FIG. 14a on the display unit 205 (steps S1203 and S1204 of FIG. 12). A guidance P1401 for the manipulation button 104 informing that a screen of the average value display process is displayed as the lower button 107 is pressed is displayed in the right side of the screen of FIG. 14A (a character string "AVERAGE" is displayed).

If the pointer 401 indicates the last record of the glucose level table 306 (step S1206 of FIG. 12), the I/O controller 307 displays the screen of FIG. 14C on the display unit 205 (steps S1207 and S1204 of FIG. 12). A guidance P1402 for the manipulation button 104 informing that the screen of the average value display process is displayed as the upper button 105 is pressed is displayed in the right side of the screen of FIG. 14C (a character string "AVERAGE" is displayed).

If the pointer 401 indicates records other than the initial record or the last record of the glucose level table 306, the I/O controller 307 displays the screen of FIG. 14b on the display unit 205 (steps S1208 and S1204 of FIG. 12).

If the upper button 105 is pressed in the screen of FIG. 14C, the pointer 401 indicates a label of "AVERAGE OF MORNING GLUCOSE LEVEL" (step S1302 of FIG. 13). In this regard, the I/O controller 307 displays the screen of FIG. 14F on the display unit 205 (step S1303 of FIG. 13).

If the upper button 105 is further pressed in the screen of FIG. 14F, the pointer 401 indicates the label of "AVERAGE OF DAYTIME GLUCOSE LEVEL" (step S1305 of FIG. 13). In this regard, the I/O controller 307 displays the screen of FIG. 14E on the display unit 205 (step S1306 of FIG. 13).

If the upper button 105 is further pressed in the screen of FIG. 14E, or the lower button 107 is further pressed in the screen of FIG. 14A, the pointer 401 indicates the label of "AVERAGE OF EVENING GLUCOSE LEVEL." In this regard, the I/O controller 307 displays the screen of FIG. 14F on the display unit 205 (step S1307 of FIG. 13).

As apparent from the aforementioned description, the memory contents display process and the average value display process are performed in a seamless manner just by manipulating the upper button 105 and the lower button 107.

### Alarm Rumbling Process

FIG. 15 is a flowchart illustrating an operation flow of the glucose meter 101 in the alarm rumbling process. FIG. 15 corresponds to step S712 of FIG. 7 and is also executed from step S1009 of FIG. 10.

As the process of step S712 of FIG. 7 starts (S1501), the I/O controller 307 causes the buzzer 211 to rumble (alarm rumbling) and sets the logic of the postprandial flag to "TRUE" (S1502). Then, the I/O controller 307 displays a screen informing that the alarm is rumbling on the display unit 205 (S1503). In addition, the I/O controller 307 checks the states of the manipulation button 104, the microswitch 210, and the RTC 204 (S1504).

First, the I/O controller 307 checks whether or not the cap 108 is removed (S1505). If the cap 108 is removed (YES in S1505), the I/O controller 307 stops the alarm (S1506) and causes the glucose meter to advance to the measurement mode (S1507).

If the cap 108 is not removed in step S1505 (NO in S1505), the I/O controller 307 checks whether or not a short press is performed for the middle button 106 (S1508). If a short press is performed for the middle button 106 (YES in S1508), the I/O controller 307 stops the alarm (S1509) and displays the time elapsing from the alarm rumbling timing on the display unit 205 (S1510). In addition, the I/O controller 307 checks the states of the manipulation button 104, the microswitch 210, and the RTC 204 (S1511).

Then, the I/O controller 307 checks whether or not a short press is performed for the middle button 106 (S1512). If a short press is performed for the middle button 106 (YES in S1512), the I/O controller 307 advances to the contents mode (S1513).

If a short press is not performed for the middle button 106 in step S1512 (NO in S1512), the I/O controller 307 executes the exception process of the contents mode (S1514) and checks the states of the manipulation button 104, the microswitch 210, and the RTC 204 (S1511).

If a short press is not performed for the middle button 106 in step S1508 (NO in S1508), the I/O controller 307 executes the exception process of the contents mode (S1515) and checks the states of the manipulation button 104, the microswitch 210, and the RTC 204 again (S1504).

### Postprandial Flag Manipulation Process and Manipulation Timer 308 Restart Process

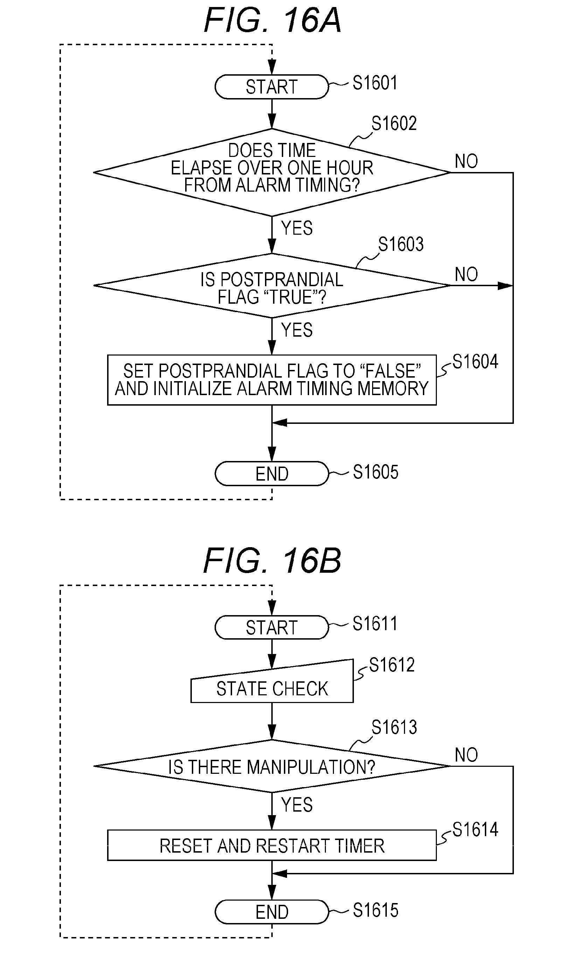

FIGS. 16A and 16B are flowcharts illustrating a process performed by the glucose meter 101 at all times under any one of the contents mode, the measurement mode, or the setup mode (that is, not the power saving mode).

FIG. 16A is a flowchart illustrating the postprandial flag manipulation process.

As the process starts (S1601), initially, the I/O controller 307 references the alarm timing stored in the alarm timing memory 309 and checks whether or not one hour or longer elapses from the alarm timing at the current time (S1602).

If one hour or longer elapses from the alarm timing at the current time (YES in S1602), the I/O controller 307 checks whether or not the postprandial flag is set to a logic value "TRUE" (S1603).

If the postprandial flag is set to the logic value "TRUE," (YES in S1603), the I/O controller 307 sets the postprandial flag to a logic value "FALSE" and initializes the alarm timing memory 309 (S1604). Then, a series of processes are terminated (S1605).

If one hour or longer does not elapse from the alarm timing at the current time in step S1602 or the alarm timing memory 309 is already initialized (NO in S1602), and if the postprandial flag is set to a logic value "FALSE" in step S1603 (NO in S1603), the I/O controller 307 terminates the process without doing anything (S1605).

The postprandial flag manipulation process is repeatedly executed at all times when the glucose meter is not under the power saving mode (from S1605 to S1601).

FIG. 16B is a flowchart illustrating a process of restarting the manipulation timer 308.

As the process starts (S1611), the I/O controller 307 checks the states of the manipulation button 104, the microswitch 210, and the glucose level measurement unit 301 (S1612). In addition, if the manipulation button 104 is manipulated, the state of the microswitch 210 changes, or the glucose level measurement unit 301 executes a glucose level measurement process (YES in S1613), the I/O controller 307 resets and restarts the manipulation timer 308 (S1614) and terminates a series of processes (S1615).

The restart process of the manipulation timer 308 is also repeatedly executed at all times while the glucose meter is not under the power saving mode (from S1615 to S1611)

### Postprandial Alarm Display Screen

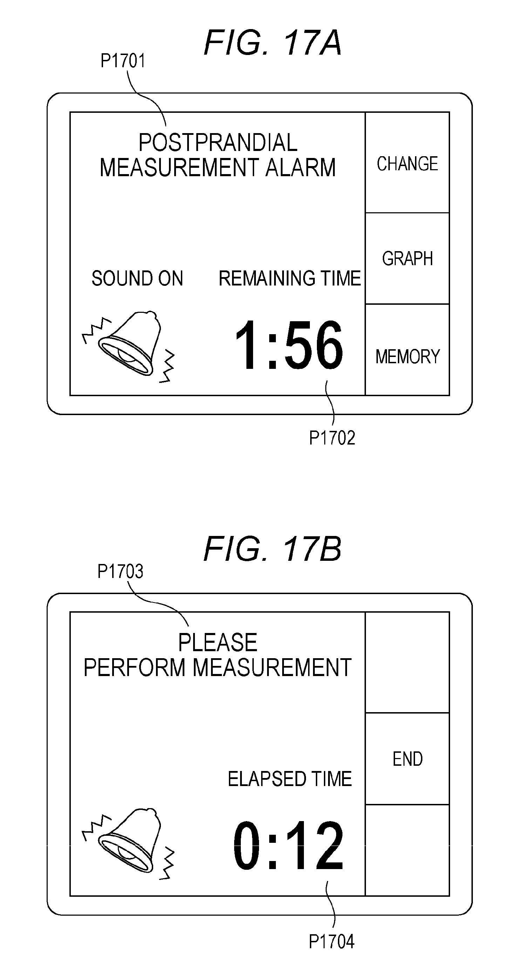

FIGS. 17A and 17B are diagrams illustrating a postprandial alarm display screen.

FIG. 17A illustrates a screen displayed on the liquid crystal display 103 (display unit 205) when two hours or shorter remains until the alarm rumbling timing at the current time. FIG. 17A illustrates a screen displayed in step S903 of FIG. 9. In the screen, a message P1701 informing a fact that the postprandial alarm is set and the remaining time P1702 until the postprandial alarm rumbling timing are displayed.

FIG. 17B illustrates a screen displayed on the liquid crystal display 103 (display unit 205) when one hour or shorter elapses after the alarm rumbling timing at the current time. FIG. 17B illustrates a screen displayed in step S906 of FIG. 9. In the screen, a message P1703 for prompting measurement of the glucose level and an elapsed time P1704 from the postprandial alarm rumbling timing are displayed.

In this manner, since the elapsed time after the postprandial alarm rumbling timing is displayed, it is possible to promote a patient's self-management will.

When the screen of FIG. 17B is displayed (YES in step S905 of FIG. 9), a screen informing a fact that the postprandial flag is set to a logic value "TRUE" (NO in step S1602 of FIG. 16) is also displayed. Therefore, a patient can apparently recognize a postprandial glucose level measurement task and smoothly perform the process.

### Setup Mode

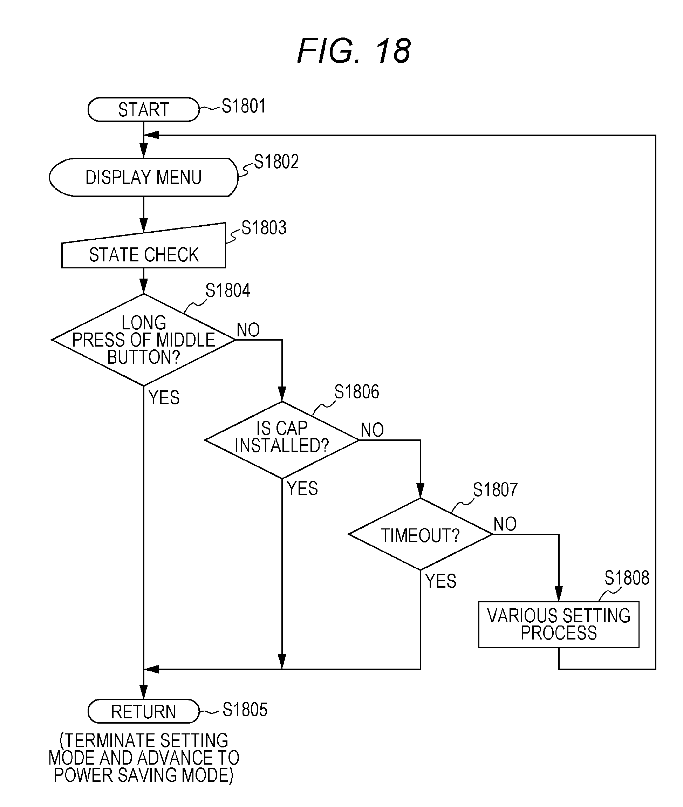

FIG. 18 is a flowchart illustrating an operation flow of the glucose meter 101 in the setup mode. FIG. 18 corresponds to step S710 of FIG. 7.

As the process starts (S1801), the I/O controller 307 initially displays a menu screen for the setup mode on the display unit 205 (S1802).

Then, the I/O controller 307 checks the states of the manipulation button 104, the microswitch 210, and the manipulation timer 308 (S1803). As a result, if a long press is performed for the middle button 106 (YES in S1804), the I/O controller 307 terminates the setup mode (S1805). As a result, the glucose meter 101 advances to the power saving mode (step S702 of FIG. 7).

If a long press is not performed for the middle button 106 in step S1804 (NO in S1804), the I/O controller 307 references the state of the microswitch 210 and checks whether or not the cap 108 is installed (S1806). If the cap 108 is installed (YES in S1806), the I/O controller 307 terminates the process of the setup mode (S1805). As a result, the glucose meter 101 advances to the power saving mode (step S702 of FIG. 7).

If the cap 108 is not installed in step S1806 (NO in S1806), the I/O controller 307 references the state of the manipulation timer 308 and checks whether or not time runs out (S1807). If time runs out (YES in S1807), the I/O controller 307 terminates the process of the setup mode (S1805). As a result, the glucose meter 101 advances to the power saving mode (step S702 of FIG. 7).

If time does not run out in the manipulation timer 308 in step S1807 (NO in S1807), the I/O controller 307 executes various setting processes based on the manipulation information output from the manipulation unit 206 (S1808) and repeats the process from step S1802 again.

### Measurement Mode

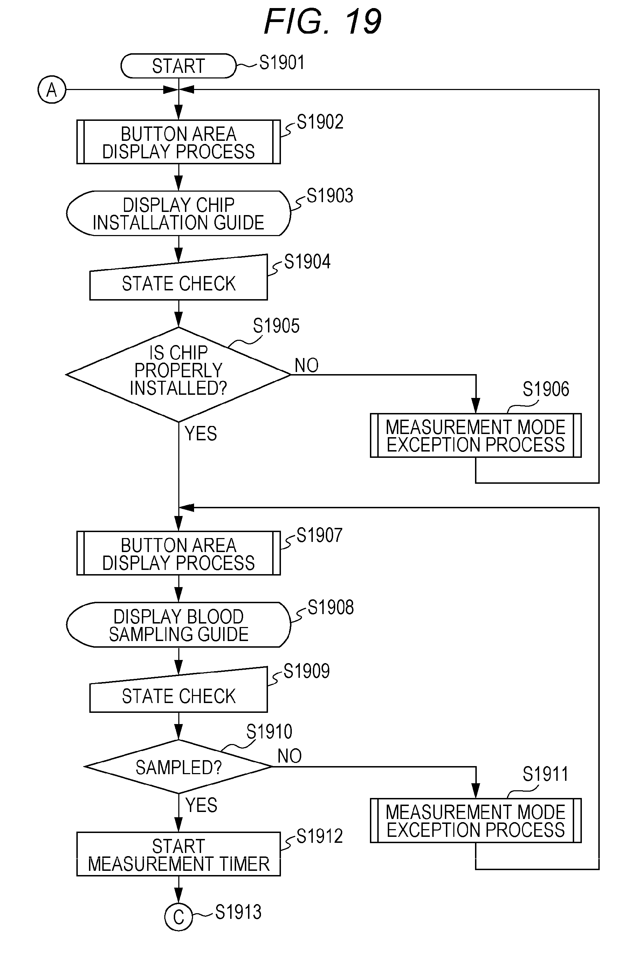

FIGS. 19 and 20 are flowcharts illustrating an operation flow of the glucose meter 101 in the measurement mode. FIGS. 19 and 20 correspond to step S708 of FIG. 7 and are also executed from step S1007 of FIG. 10.

As the process of step S708 of FIG. 7 starts (S1901), initially, the I/O controller 307 executes a button area display process for displaying a button area neighboring to the manipulation button 104 on the screen of the display unit 205 (S1902). Then, a chip installation guide screen for notifying a patient of chip installation in the glucose meter 101 is displayed (S1903) as an initial step of the glucose level measurement task.

The I/O controller 307 checks the states of the manipulation button 104, the microswitch 210, the manipulation timer 308, and the photodiode 215 (S1904). As a result, if it is not detected that the chip is normally installed in the glucose meter 101 (NO in S1905), the I/O controller 307 executes the exception process of the measurement mode (S1906) and repeats the process from step S1902 again.

If it is detected that the chip is normally installed in the glucose meter 101 in step S1905 (YES in S1905), the I/O controller 307 advances to a process of sampling blood 304 on a chip which is the next step of the glucose level measurement task.

First, similar to step S1902, the button area display process is executed (S1907). Then, a blood sampling guide screen is displayed (S1908) for guiding a patient to sample blood 304 on a chip, which is the next step of the glucose level measurement task.

The I/O controller 307 checks the states of the manipulation button 104, the microswitch 210, the manipulation timer 308, and the photodiode 215 (S1909). As a result, if it is not detected that blood 304 is normally sampled on a chip (NO in S1910), the I/O controller 307 executes the exception process of the measurement mode (S1911) and repeats the process from step S1907 again.

If it is detected that blood 304 is normally sampled on the chip in step S1910 (YES in S1910), the I/O controller 307 advances to a process of measuring the glucose level which is the next step of the glucose level measurement task.

First, the I/O controller 307 starts the measurement timer 303 for measuring the glucose level (S1912). The measurement timer 303 counts, for example, 9 seconds. Then, the process continues to FIG. 20 (S1913).

The flowchart will be continuously described with reference to FIG. 20.

Then, similar to steps S1902 and S1907, the I/O controller 307 executes the button area display process (S2014). Then, the glucose level measurement task for a patient is completed, which is the next step of the glucose level measurement task. That is, a measurement in-progress guide screen is displayed for informing that the measurement timer 303 is in progress of a countdown (9 seconds in this embodiment) and guiding a patient to wait for the end of the countdown (S2015).

The I/O controller 307 checks the states of the manipulation button 104, the microswitch 210, the measurement timer 303, and the manipulation timer 308 (S2016). As a result, if the measurement timer 303 does not complete a countdown (NO in S2017), the I/O controller 307 checks whether or not a short press is performed for the upper button 105 (S2018).

If a short press is performed for the upper button 105 (YES in S2018), the I/O controller 307 sets the postprandial flag state to a logic value "TRUE" (S2019) and repeats the process from step S2014 again. That is, the I/O controller 307 sets the postprandial flag state to a logic value indicating a postprandial glucose level.

If a short press is not performed for the upper button 105 in step S2018 (NO in S2018), the I/O controller 307 checks whether or not a long press is performed for the upper button 105 (S2020).

If a long press is performed for the upper button 105 (YES in S2020), the I/O controller 307 sets the postprandial flag state to a logic value "FALSE" (S2021) and repeats the process from step S2014 again. That is, the I/O controller 307 sets the postprandial flag state to a logic value indicating a postprandial glucose level.

If a long press is not performed for the upper button 105 in step S2020 (NO in S2020), the I/O controller 307 repeats the process from step S2014 again.

If the measurement timer 303 completes a countdown in step S2017 (YES in S2017), the I/O controller 307 causes the glucose level computation unit 302 to compute the glucose level (S2022) .

Then, similar to steps S1902, S1907, and S2014, the I/O controller 307 executes the button area display process (S2023). Then, the I/O controller 307 executes the glucose level display process for displaying the glucose level on the display unit 205 (S2024). In addition, the I/O controller 307 checks the states of the manipulation button 104, the microswitch 210, and the manipulation timer 308 (S2025).

Then, the I/O controller 307 executes the glucose level recording process in which the glucose level computed in step S2022 is recorded on the glucose level table 306 along with the postprandial flag (S2026). In addition, the exception process of the measurement mode is executed (S2027).

After the glucose level recording process of step S2026 and the measurement mode exception process of step S2027 are executed in this manner, the I/O controller 307 checks whether or not the manipulation information obtained from the manipulation button 104 represents a manipulation for advancing to the contents mode (S2028). If the manipulation is for advancing to the contents mode (YES in S2028), the I/O controller 307 advances to the contents mode (S2029).

If the manipulation information obtained from the manipulation button 104 is not a manipulation for advancing to the contents mode in step S2028 (NO in S2028), the I/O controller 307 repeats the process from step S2023 again.

### Measurement Mode: Exception Process

FIG. 21 is a flowchart illustrating an operation flow of the glucose meter 101 in the exception process under the measurement mode. FIG. 21 corresponds to steps S1906 and S1911 of FIG. 19 and step S2025 of FIG. 20.

As the process starts (S2101), the I/O controller 307 checks whether or not a long press is performed for the middle button 106 (S2102). If a long press is performed for the middle button 106 (YES in S2102), the I/O controller 307 terminates the process of the measurement mode (S2103). As a result, the glucose meter 101 advances to the power saving mode (step S702 of FIG. 7).

If a long press is not performed for the middle button 106 in step S2102 (NO in S2102), the I/O controller 307 references the state of the microswitch 210 and checks whether or not the cap 108 is installed (S2104). If the cap 108 is installed (YES in S2104), the I/O controller 307 terminates the process of the measurement mode (S2103). As a result, the glucose meter 101 advances to the power saving mode (step S702 of FIG. 7).

If the cap 108 is not installed in step S2104 (NO in S2104), the I/O controller 307 references the manipulation timer 308 and checks whether or not the manipulation timer 308 reaches a timeout (S2105). If the manipulation timer 308 reaches a timeout (YES in S2105), the I/O controller 307 terminates the process of the measurement mode (S2103). As a result, the glucose meter 101 advances to the power saving mode (step S702 of FIG. 7).

If the manipulation timer 308 does not reach a timeout in step S2105 (NO in S2105), the I/O controller 307 checks whether or not the manipulation information obtained from the manipulation button 104 represents a manipulation for advancing to the contents mode (S2106). If a manipulation is made for advancing to the contents mode (YES in S2106), the I/O controller 307 advances to the contents mode (S2107).

If the manipulation information obtained from the manipulation button 104 does not represent a manipulation for advancing to the contents mode in step S2106 (NO in S2106), the I/O controller 307 checks whether or not a short press is performed for the upper button 105 (S2108).

If a short press is performed for the upper button 105 (YES in S2108), the I/O controller 307 sets the state of the postprandial flag to a logic value "TRUE" (S2109). That is, the I/O controller 307 sets the postprandial flag state to a logic value representing a postprandial glucose level. In addition, the I/O controller 307 terminates the exception process of the contents mode (S2110).

If a short press is not performed for the upper button 105 in step S2108 (NO in S2108), the I/O controller 307 checks whether or not a long press is performed for the upper button 105 (S2111).

If a long press is performed for the upper button 105 (YES in S2111), the I/O controller 307 sets the postprandial flag state to a logic value "FALSE" (S2112). That is, the I/O controller 307 sets the postprandial flag state to a logic value representing a postprandial glucose level. In addition, I/O controller 307 terminates the exception process of the contents mode (S2110).

If a long press is not performed for the upper button 105 in step S2111 (NO in S2111), the I/O controller 307 terminates the exception process of the contents mode (S2110).

### Measurement Mode: Glucose Level Recording Process

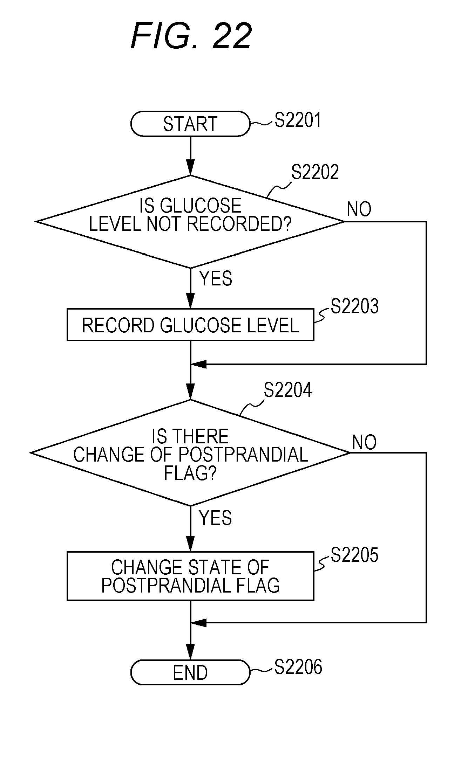

FIG. 22 is a flowchart illustrating an operation flow of the glucose meter 101 in the glucose level recording process under the measurement mode. FIG. 22 corresponds to step S2024 of FIG. 19.

As the process starts (S2201), the I/O controller 307 checks whether or not the glucose level computed in step S2020 of FIG. 20 has been recorded on the glucose level table 306 (S2202). If it is checked that the glucose level has not been recorded (YES in S2202), the I/O controller 307 records the computed glucose level on the glucose level table 306 along with the measurement date/time and the postprandial flag (S2203).

If it is checked that the glucose level has been recorded in step S2202 (NO in S2202), the I/O controller 307 does not record the computed glucose level on the glucose level table 306.

Then, the I/O controller 307 checks whether or not the logic value of the current postprandial flag matches the postprandial flag recorded on the glucose level table 306, that is, whether or not there is a change in the postprandial flag (S2204). If it is checked that there is a change in the postprandial flag (YES in S2204), the I/O controller 307 writes the logic value of the current postprandial flag to the postprandial flag field of the glucose level table 306 where the current glucose level is recorded (S2205).

If it is not checked that there is a change in the postprandial flag in step S2204 (NO in S2204), the I/O controller 307 does not record the postprandial flag on the glucose level table 306.

Even when the postprandial flag is recorded on the glucose level table 306 (S2205), or otherwise (NO in S2204), the I/O controller 307 terminates a series of processes (S2206).

### Measurement Mode: Button Area Display Process

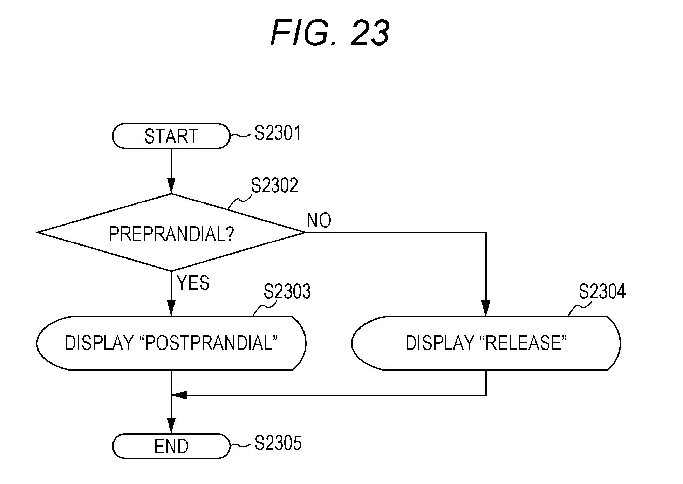

FIG. 23 is a flowchart illustrating an operation flow of the glucose meter 101 in the button area display process under the measurement mode. FIG. 23 corresponds to steps S1902 and S1907 of FIG. 19 and steps S2014 and S2023 of FIG. 20.

As the process starts (S2301), the I/O controller 307 references the postprandial flag and checks whether or not the logic value of the postprandial flag represents "preprandial" (S2302).

If the logic value of the postprandial flag represents "preprandial" (YES in S2302), the I/O controller 307 displays a character string "POSTPRANDIAL" in a button area located in the right end of the display unit 205 neighboring to the upper button 105 (S2303).

If the logic value of the postprandial flag represents "postprandial" (NO in S2302), the I/O controller 307 displays a character string "RELEASE" in a button area located in the right end of the display unit 205 neighboring to the upper button 105 (S2304).

In either case of step S2303 or S2304, a series of processes are terminated (S2305) thereafter.

### Contents Mode and Measurement Mode: Glucose Level Display Process

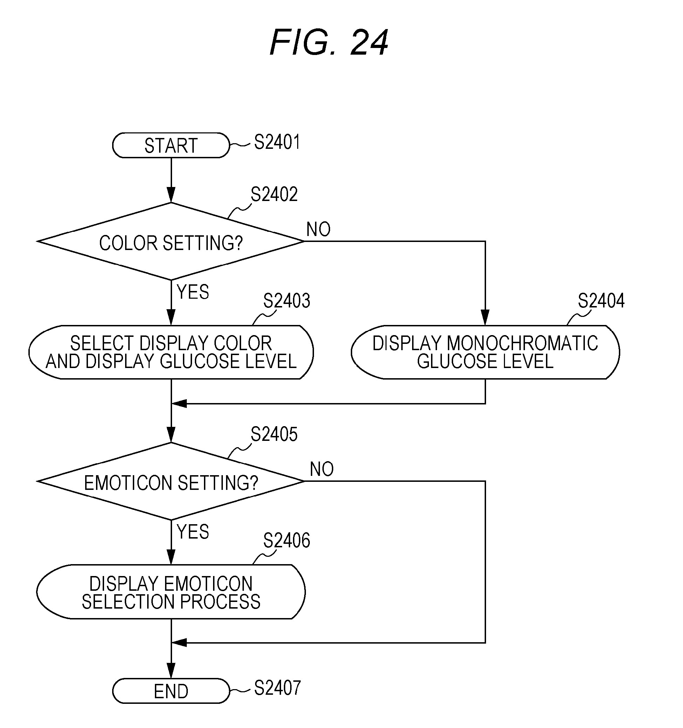

FIG. 24 is a flowchart illustrating an operation flow of the glucose meter 101 in the glucose level display process under the contents mode and the measurement mode. FIG. 24 corresponds to step S1204 of FIG. 12 and step S2022 of FIG. 20.

As the process starts (S2401), the I/O controller 307 references miscellaneous setup information and checks whether or not a color display is set in the setup information of the screen displayed on the display unit 205 (S2402).

If a color display is set in the setup information of the screen displayed on the display unit 205 (YES in S2402), the I/O controller 307 compares the glucose level to be displayed with a predetermined threshold value and performs 5-grade evaluation. In addition, the I/O controller 307 selects a display color based on a result of 5-grade evaluation and displays the glucose level on the display unit 205 (S2403).

If a monochromatic display is set in the setup information of the screen displayed on the display unit 205 (NO in S2402), the I/O controller 307 displays, on a monochromatic screen of the display unit 205, the glucose level to be displayed (S2404) .

In either case of step S2403 or S2404, the I/O controller 307 references miscellaneous setup information and checks whether or not an emoticon display is set in the setup information of the screen displayed on the display unit 205 (S2405).

If it is checked that an emoticon display is set in the setup information of the screen displayed on the display unit 205 (YES in S2405), the I/O controller 307 compares the glucose level to be displayed with a predetermined threshold value and performs 5-grade evaluation. In addition, I/O controller 307 selects an emoticon based on a result of the 5-grade evaluation and displays the glucose level on the display unit 205 (S2406) .

If it is checked that an emoticon display is not set in the setup information of the screen displayed on the display unit 205 (NO in S2405), the I/O controller 307 does not display an emoticon.

In either case of step S2406 or S2405, the I/O controller 307 terminates a series of processes thereafter (S2407).

### Measurement Mode: Display Screen in Glucose Level Display Process

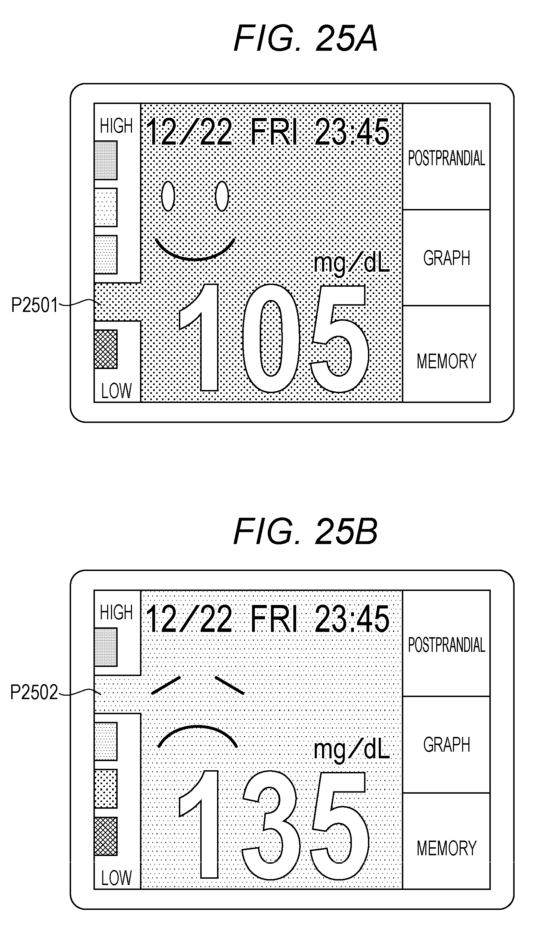

FIGS. 25A and 25B illustrate a display screen displayed on the display unit 205 of the glucose meter 101 in the glucose level display process under the measurement mode. More specifically, FIGS. 25A and 25B illustrate a display screen displayed in steps S2403 and S2406 of FIG. 24.

If a color display is set in miscellaneous setup information, the glucose level is evaluated into 5 classifications. The lowest glucose level is displayed as a blue color, the second lowest glucose level as an optimal glucose level is displayed as a yellow-green color, the middle glucose level is displayed as an orange color, the second highest glucose level is displayed as a pink color, and the highest glucose level is displayed as a red color.

FIG. 25A illustrates a screen displayed for the second lowest glucose level out of the 5-grade evaluation. This display screen is displayed as a yellow-green color corresponding to an optimal glucose level and is integrated into a yellow-green portion located in the second level from the bottom in the classification scale displayed in the left end. By integrating the classification scale and the background portion of the screen, a tab P2501 is formed on the display screen.

FIG. 25B illustrates a screen displayed for the second highest glucose level out of the 5-grade evaluation. This display screen is displayed as a pink color corresponding to a second highest glucose level and is integrated into a pink portion located in the second level from the top in the classification scale displayed in the left end so that a tab P2502 is formed.

In this manner, since a tab is formed by integrating the classification scale and the background of the screen, it is possible to apparently and immediately recognize the current glucose level in the 5-grade evaluation.

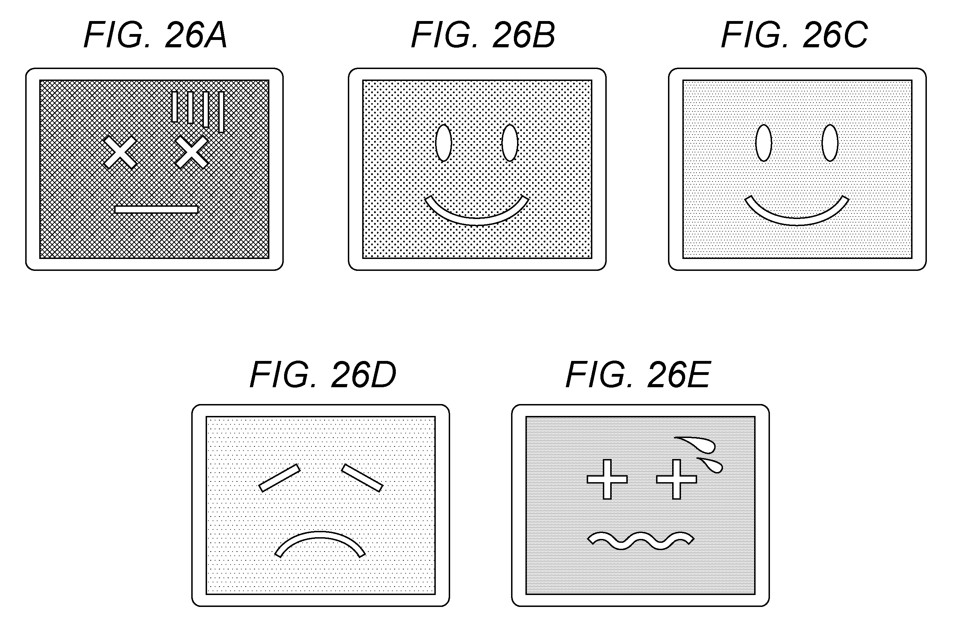

FIGS. 26A to 26E are diagrams illustrating a variation of the emoticon displayed on the display unit 205 in the process of displaying the glucose level using the glucose meter 101 under the contents mode and the measurement mode. They are emoticons displayed on the display unit 205 depending on the glucose level when an emoticon display is set in the setup information of the screen displayed on the display unit 205 in step S2405 of FIG. 24 (YES in S2405).

The I/O controller 307 compares the glucose level to be displayed with a predetermined threshold value and performs 5-grade evaluation. In addition, the I/O controller 307 selects an emoticon based on a result of the 5-grade evaluation and displays the glucose level on the display unit 205 (S2406).

The 5-grade evaluation is performed using threshold values conforming to Japan Diabetes Study, "diabetes care guideline (http://www.lifescience.jp/ebm/cms/ms/no.19/topics.pdf)", and emoticons of FIGS. 26A to 26E are displayed based on the 5-grade evaluation.

FIG. 26A illustrates an emoticon representing a low glucose level. This emoticon is displayed when a fasting glucose level and a postprandial glucose level after 2 hours are lower than 80 mg/dL.

FIG. 26B illustrates an emoticon representing an adequate glucose level. This emoticon is displayed when a fasting glucose level is 80 to 110 mg/dL, and the postprandial glucose level after 2 hours is 80 to 140 mg/dL.

FIG. 26C illustrates an emoticon representing that the glucose level is slightly high. This emoticon is displayed when a fasting glucose level is equal to or higher than 110 mg/dL and lower than 130 mg/dL, and the postprandial glucose level after 2 hours is equal to or higher than 140 mg/dL and is lower than 180 mg/dL.

FIG. 26D illustrates an emoticon representing that the glucose level is high. This emoticon is displayed when a fasting glucose level is equal to or higher than 130 mg/dL and is lower than 160 mg/dL, and the postprandial glucose level after 2 hours is equal to or higher than 180 mg/dL and is lower than 220 mg/dL.

FIG. 26E illustrates an emoticon representing that the glucose level is too high and dangerous. This emoticon is displayed when a fasting glucose level is equal to or higher than 160 mg/dL, and the postprandial glucose level after 2 hours is equal to or higher than 220 mg/dL.

In this manner, the emoticons having different faces illustrated in FIGS. 26A to 26E correspond to glucose levels. That is, in the glucose meter 101 according to this embodiment, the glucose level is applied to an emoticon having a different face and is displayed on the display unit 205. Therefore, it is possible to immediately and apparently recognize the current glucose level in the 5-grade evaluation.

### Contents Mode and Measurement Mode: Display Screen

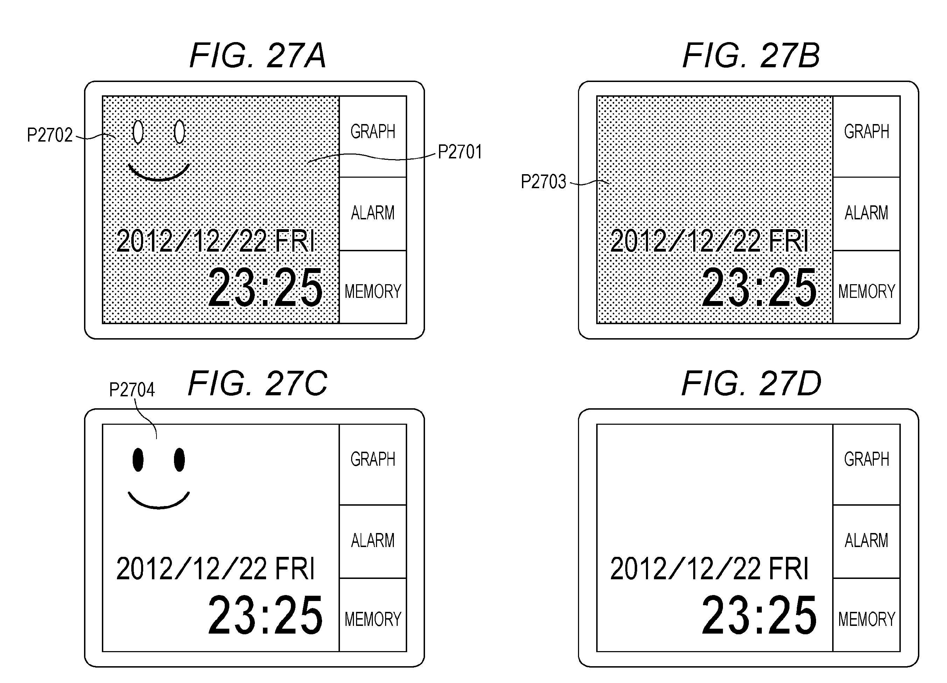

FIGS. 27A to 27D are diagrams illustrating a variation of the display screen displayed on the display unit 205 in a process of displaying the glucose level using the glucose meter 101 under the contents mode and the measurement mode. More specifically, FIGS. 27A to 27D illustrate display screens displayed in step S1204 of FIG. 12.

FIG. 27A illustrates a display screen displayed when a color setting (YES in S2402) and an emoticon setting (YES in S2405) are used in the miscellaneous setup information. On the screen, both the background color P2701 based on the 5-grade evaluation and the emoticon P2702 based on the 5-grade evaluation are displayed.

FIG. 27B illustrates a display screen displayed when a color setting (YES in S2402) and no emoticon setting (NO in S2405) are used in the miscellaneous setup information. On the screen, the background color P2703 based on the 5-grade evaluation is displayed, but the emoticon P2702 based on the 5-grade evaluation is not displayed.

FIG. 27C illustrates a display screen displayed when a monochromatic setting (YES in S2402) and an emoticon setting (YES in S2405) are used in the miscellaneous setup information. On the screen, a monochromatic background is displayed, and the emoticon P2704 based on the 5-grade evaluation is displayed.

FIG. 27D illustrates a display screen displayed when a monochromatic setting (YES in S2402) and no emoticon setting (NO in S2405) are used in the miscellaneous setup information. On the screen, a monochromatic background is displayed, but the emoticon based on the 5-grade evaluation is not displayed.

In this manner, a status of the screen displayed on the display unit 205 changes depending on the settings of the miscellaneous setup information.

It is noted that, although FIGS. 27A to 27D illustrate a variation of the display screen under the contents mode, a variation of the display screen may also be possible under the measurement mode.

The color display made in consideration of a patient's convenience may not be visible, depending on a combination of colors, to a patient who suffers from a visual disorder caused by complications such as diabetic retinopathy. For this reason, if a function of switching to a high-contrast display such as a monochromatic display and a function of switching between a mark display and a no-mark display are provided in the glucose meter 101, it is possible to allow a patient to select an optimal glucose level display matching a visual disorder degree on the display unit 205.

### Measurement Mode Postprandial Button and Contents Mode Postprandial Alarm Button

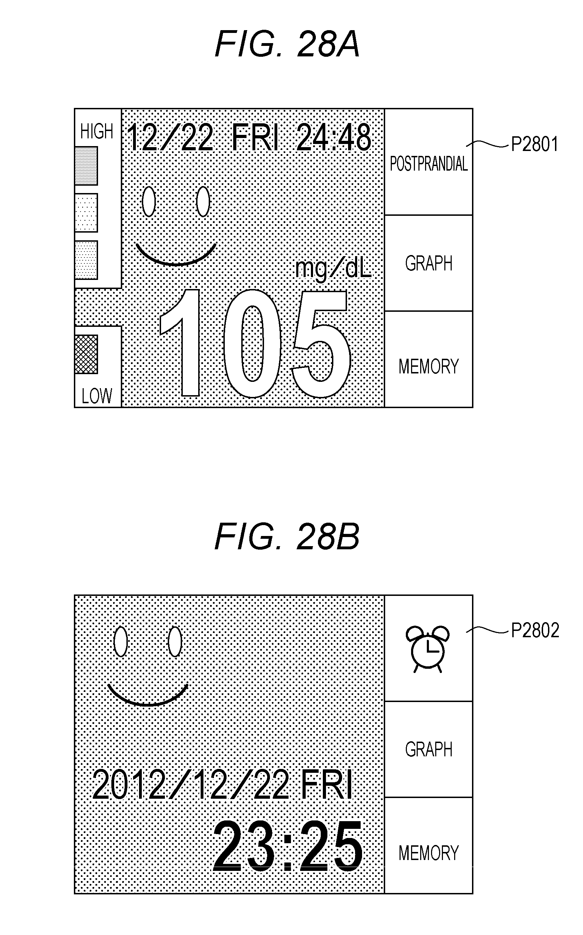

FIGS. 28A and 28B are display screens under the measurement mode and the contents mode.

In both of FIGS. 28A and 28B, a button display area is provided in the right end of the screen in order to describe functions of the manipulation button 104 arranged in the vicinity thereof.

In the button display area of FIG. 28A, a character string "POSTPRANDIAL" is displayed in the area P2801 of the upper button 105. The upper button 105 serves as a postprandial button under the measurement mode, and the postprandial flag changes as a short press is performed for the upper button 105.

In the button display area of FIG. 28B, a timepiece is illustrated in the area P2802 of the upper button 105. The upper button 105 under the contents mode has a function of setting the postprandial alarm. If a short press is performed for the upper button 105, a menu screen for setting the postprandial alarm is displayed.

### Function Allocation of Manipulation Button 104

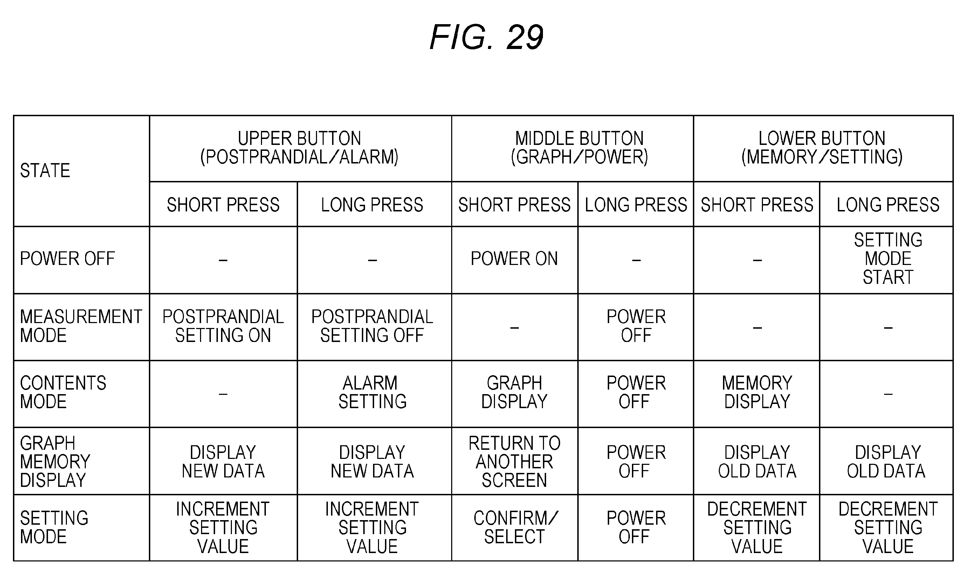

FIG. 29 is a table showing functions allocated to the manipulation buttons 104.

Hereinbefore, main functions allocated to the manipulation buttons 104 have been described with reference to the flowcharts of FIGS. 7 to 24. The table of FIG. 29 shows a matching relationship between the manipulation buttons 104 and the functions allocated to each operation mode.

In the power-off state, that is, in the power saving mode state, the I/O controller 307 detects only a short press of the middle button 106 and a long press of the lower button 107.

If a short press is performed for the middle button 106, the I/O controller 307 advances to the contents mode. That is, the glucose meter has a power-on state.

If a long press is performed for the lower button 107, the I/O controller 307 advances to the setup mode.

Although not shown in this table, removal of the cap 108 is also detected using the microswitch 210. If the cap 108 is removed, the I/O controller 307 advances to the measurement mode.

In the measurement mode state, the I/O controller 307 detects only a short press and a long press of the upper button 105 and a long press of the middle button 106.

If a short press is performed for the upper button 105, the I/O controller 307 sets the postprandial flag to a logic value "TRUE." That is, the postprandial setting is turned on.

If a long press is performed for the upper button 105, the I/O controller 307 sets the postprandial flag to a logic value "FALSE." That is, the postprandial setting is turned off.

If a long press is performed for the middle button 106, the I/O controller 307 advances to the power saving mode. That is, the glucose meter has a power-off state.

In the contents mode state, the I/O controller 307 detects only a long press of the upper button 105, a short press and a long press of the middle button 106, and a short press of the lower button 107.

If a long press is performed for the upper button 105, the I/O controller 307 activates the alarm setup function.

If a short press is performed for the middle button 106, the I/O controller 307 activates a graph display function.

If a long press is performed for the middle button 106, the I/O controller 307 advances to the power saving mode. That is, the glucose meter has a power-off state.

If a short press is performed for the lower button 107, the I/O controller 307 activates a memory display function.

When the graph display function and the memory display function are activated, the I/O controller 307 detects a short press and a long press of the upper button 105, a short press and a long press of the middle button 106, and a short press and a long press of the lower button 107, that is, a short press and a long press of overall buttons.

If a short press is performed for the upper button 105, the I/O controller 307 shifts the pointer 401 indicating currently displayed data to new data and displays the new data on the display unit 205.

If a long press is performed for the upper button 105, the I/O controller 307 shifts the pointer 401 indicating the currently displayed data to new data and displays the new data on the display unit 205. It is noted that, in this manipulation, the I/O controller 307 continuously displays the data on the display unit 205 as long as possible.

If a short press is performed for the middle button 106, the I/O controller 307 returns the screen to the previous one used to call the currently executed function.

If a long press is performed for the middle button 106, the I/O controller 307 advances to the power saving mode. That is, the glucose meter has a power-off state.

If a short press is performed for the lower button 107, the I/O controller 307 shifts the pointer 401 indicating the currently displayed data to old data and displays the old data on the display unit 205.

If a long press is performed for the lower button 107, the I/O controller 307 shifts the pointer 401 indicating the currently displayed data to old data and displays the old data on the display unit 205. It is noted that, in this manipulation, the I/O controller 307 continuously displays the data on the display unit 205 as long as possible.

In the setup mode state, the I/O controller 307 detects a short press and a long press of the upper button 105, a short press and a long press of the middle button 106, and a short press and a long press of the lower button 107, that is, a short press and a long press of overall buttons.

If a short press is performed for the upper button 105, the I/O controller 307 increments the currently manipulated setup value ("+1").

If a long press is performed for the upper button 105, the I/O controller 307 continuously increments the currently manipulated setup value.

If a short press is performed for the middle button 106, the I/O controller 307 determines the currently investigated setup value or selects one of a plurality of options.

If a long press is performed for the middle button 106, the I/O controller 307 advances to the power saving mode. That is, the glucose meter has a power-off state.

If a short press is performed for the lower button 107, the I/O controller 307 decrements the currently manipulated setup value ("-1").

If a long press is performed for the lower button 107, the I/O controller 307 continuously decrements the currently manipulated setup value.

According to this embodiment, a glucose meter 101 is disclosed.

A cap 108 is provided to protect an optical measurement unit 109, and a microswitch 210 is provided to detect an installation state of the cap 108 in the casing 102. If the I/O controller 307 detects that the cap 108 is removed from the casing 102, the glucose meter advances from the power saving mode to the measurement mode. In this manner, by using the cap 108 as a sort of the manipulation button 104, it is possible to provide a glucose meter 101 having an easy-understanding manipulation system for a patient using a minimum number of manipulation buttons 104.

In the glucose meter 101, a postprandial alarm function is provided to inform a patient of a fact that it is the postprandial glucose level measurement timing. If it is detected that the current time obtained from the RTC 204 reaches the alarm rumbling timing stored in the alarm timing memory 309, the I/O controller 307 causes the alarm (buzzer 211) to rumble and sets the postprandial flag of the postprandial flag memory 310 to a logic value "TRUE" for a predetermined time after the alarm rumbling timing. In addition, the postprandial flag is recorded on the glucose level table 306 along with the glucose level measured by the glucose level measurement unit 301. In this manner, since the postprandial alarm and the postprandial flag are operated together, it is possible to allow a patient to more reliably perform a postprandial glucose level measurement task.

Three manipulation buttons 104 including the upper button 105, the middle button 106, and the lower button 107 are provided in the glucose meter 101. In the measurement mode in which the glucose level is measured, the upper button 105 is used to manipulate the postprandial flag indicating whether or not the measurement glucose level recorded on the glucose level table 306 is a postprandial glucose level. Meanwhile, in the contents mode in which the glucose level recorded on the glucose level table 306 is referenced, the upper button 105 is used to set the postprandial alarm function for informing a patient of a fact that it is the postprandial glucose level measurement time. In this manner, a function relating to a concept of "postprandial" is allocated to a single manipulation button, that is, the upper button 105 when the glucose level is measured and reviewed. Therefore, it is possible to provide a glucose meter allowing a patient to easily memorize how to manipulate the glucose meter and having both high performance and simplicity.

Three manipulation buttons 104 including the upper button 105, the middle button 106, and the lower button 107 are provided in the glucose meter 101. If the lower button 107 is continuously pressed under the contents mode in which the glucose level recorded on the glucose level table 306, a pointer reaches a starting end record of the glucose level table 306. If the lower button 107 is further pressed, a recent one-moth average of the morning glucose levels stored in the glucose level table 306 is displayed. That is, it is possible to switch between the glucose level display function and the average value display function in a seamless manner just by shifting the reference record. Therefore, it is possible to allow a patient to easily memorize how to manipulate the glucose meter without a necessity of a cumbersome manipulation such as a mode switching.

The glucose level measured by the glucose level measurement unit 301 of the glucose meter 101 is compared with a plurality of threshold values to perform 5-grade evaluation. Matching the 5-grade evaluation result, five colors are allocated to display a corresponding color of the glucose level on the liquid crystal display 103. In addition, a 5-color scale is displayed in one end of the liquid crystal display 103, and a background color matching the corresponding scale is integrated into the scale and is displayed like a tab. In this manner, using a tab-like display, it is possible to recognize the measurement glucose level at a glance using a position of the tab.

The I/O controller 307 controls a display state of the liquid crystal display 103 based on a color setting and an emoticon setting stored in the miscellaneous setup information memory 311. If the color display is set, the glucose level measured by the glucose level measurement unit 301 is compared with a plurality of threshold values to perform 5-grade evaluation, and five colors are allocated to the evaluation result so that a color corresponding to the glucose level is displayed on the liquid crystal display 103. If the emoticon display is set, an emoticon corresponding to the 5-grade evaluation result is displayed on the liquid crystal display 103. In this manner, a setup function for displaying the glucose meter 101 is set precisely. Therefore, it is possible to implement an optimal glucose level display function depending on a patient preference or considering whether or not a patient suffers from a visual disorder.

Three manipulation buttons 104 including the upper button 105, the middle button 106, and the lower button 107 are provided in the glucose meter 101. In the measurement mode in which a glucose level is measured, a function of manipulating the postprandial flag indicating whether or not the measurement glucose level recorded on the glucose level table 306 is a postprandial glucose level is allocated to the upper button 105. Meanwhile, in the contents mode in which the glucose level recorded on the glucose level table 306 is referenced, a function of setting the postprandial alarm for informing a patient of a fact that it is the postprandial glucose level measurement time is allocated to the upper button 105. A power-off function is allocated to the middle button 106 in either the measurement mode or the contents mode. In this manner, it is possible to implement a lot of functions using a minimum number of manipulation buttons by allocating different functions to a small number of manipulation buttons depending to the operation mode.

While the embodiments of the invention have been described hereinbefore, they are not intended to limit the invention. Various modifications or application may be possible without departing from the spirit and scope of the invention as described in the appended claims.

A glucose meter has a postprandial alarm function for informing a patient of a fact that it is the postprandial glucose level measurement time. As it is detected that the current time obtained from an RTC reaches an alarm rumbling timing stored in an alarm timing memory, the I/O controller causes an alarm (buzzer) to rumble and sets a postprandial flag of a postprandial flag memory to a logic value "TRUE" for a predetermined time after the alarm rumbling timing. The postprandial flag is recorded on the glucose level table along with the glucose level measured by a glucose level measurement unit.

## Claims

1. A glucose meter comprising:
a measurement unit where a test strip for measuring a glucose level of a patient is installed;
a glucose level measurement unit that obtains a predetermined signal from the measurement unit and measures a glucose level of the patient;
a glucose level table including a date/time field where date/time information regarding a date and a time for measuring the glucose level is recorded, a glucose level field where the measured glucose level is recorded, and a mealtime flag field where a mealtime flag for identifying whether the measured glucose level is a preprandial glucose level or a postprandial glucose level is recorded;
a time counting unit that generates the date/time information;
an alarm timing memory that stores an alarm setting timing;
a display unit that displays information representing a fact that it is the alarm setting timing when a current time obtained from the time counting unit matches the alarm setting timing; and
an I/O controller that records a logic value indicating a fact that the measured glucose level is a postprandial glucose level on the mealtime flag field when the glucose level measurement unit records the measured glucose level on the glucose level table as long as the current time is within a predetermined period of time from the alarm setting timing.

2. The glucose meter according to claim 1, wherein the I/O controller displays an elapsed time from the alarm setting timing on the display unit when the current time is within a predetermined period of time from the alarm setting timing.
